(19) Europäisches Patentamt
European Patent Office
Office européen des brevets

(11) Veröffentlichungsnummer : **0 376 889 B1**

# (12) EUROPÄISCHE PATENTSCHRIFT

(45) Veröffentlichungstag der Patentschrift : **19.05.93 Patentblatt 93/20**

(21) Anmeldenummer : **89810974.9**

(22) Anmeldetag : **21.12.89**

(51) Int. Cl.[5] : **C07C 333/10,** C07C 333/26, C07C 335/24, C07C 335/26, C10M 135/16, C10M 135/18, C10M 135/26

## (54) Schmierstoffzusammensetzung.

(30) Priorität : **28.12.88 CH 4828/88**

(43) Veröffentlichungstag der Anmeldung : **04.07.90 Patentblatt 90/27**

(45) Bekanntmachung des Hinweises auf die Patenterteilung : **19.05.93 Patentblatt 93/20**

(84) Benannte Vertragsstaaten : **BE DE ES FR GB IT NL**

(56) Entgegenhaltungen :
**EP-A- 0 000 514**
**DE-A- 1 443 886**
**US-A- 4 659 853**
**JOURNAL FÜR PRAKTISCHE CHEMIE, Band 315, Heft 1, 1973, Seiten 144-148, Leipzig, DD; H. HARTMANN et al.: "Darstellung und Charakterisierung 1,1-disubstituierter Thioharnstoffe"**
**PHARMAZEUTISCHE ZENTRALHALLE, Band 107, Heft 7, 1968, Seiten 493-500, Jena, DE; E. SCHRÖPL et al.: "Zur Kenntnis von Säureisothiocyanaten. Umsetzung der Säureisothiocyanate mit aliphatischen Alkoholen und Thioalkoholen zu N-Acylthiourethanen und N-Acyldithiourethanen"**
**CHEMICAL ABSTRACTS, Band 78, Heft 7, 19. Februar 1973, Seite 485, Zusammenfassung Nr. 43396, Columbus, Ohio, US; N.M. TURKEVICH et al.: "1,3-Thiazan-4-ones"**
**CHEMISCHE BERICHTE, Band 120, 1987, Seiten 1251-1253, VCH Verlagsgesellschaft mbH, Weinheim, DE; K.-H. KÖNIG et al.: "Symmetrische 3,3,3',3'-Tetraalkyl-1,1'-alkandioylbis(thioharnstoffe) als neue Chelatliganden"**
**SYNTHESIS, Band 1985, April 1985, Seiten 423-426, Stuttgart, DE; L.L. WHITFIELD et al.: "Preparation and reactions of methyl 2-(isothiocyanatocarbonyl)-benzoate: Synthesis of derivatives of [1,2,4]triazolo[5,1-alpha]isoquinoline"**

(56) Entgegenhaltungen :
**J. CHEM. SOC. PERKIN TRANS. 1, Band 1987, 1987, Seiten 1153-1158, Letchworth, GB; J.C. BRINDLEY et al.: "N'-substituted N-acyl- and N-imidoyl-thioureas: Preparation and conversion of N',N'-disubstituted compounds into 2-(N,N-disubstituted amino)thiazol-5-yl ketones"**

(73) Patentinhaber : **CIBA-GEIGY AG**
**Klybeckstrasse 141**
**CH-4002 Basel (CH)**

(72) Erfinder : **Camenzind, Hugo, Dr.**
**Av. Général Guisan 42**
**CH-1700 Fribourg (CH)**
Erfinder : **Nesvadba, Peter, Dr.**
**Route du Nord 5**
**CH-17243 Marly (CH)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue Saint-Denis, 75001 PARIS

## Beschreibung

Vorliegende Erfindung betrifft neue Zusammensetzungen, enthaltend einen Schmierstoff oder eine Hydraulikflüssigkeit, und wenigstens eine Verbindung, die als Verschleissschutz für Metallteile, die einer Reibabnutzung unterliegen, und als Antioxidans in Schmierstoffen wirkt. Ferner betrifft vorliegende Erfindung neue Verbindungen und die Verwendung der Zusammensetzungen und neuen Verbindungen.

Es ist bekannt, Schmierstoffen z.B. wie Schmierölen auf mineralischer und/oder synthetischer Basis, Additive zuzugeben, die beispielsweise als Verschleissschutz für Metallteile wirken und somit den Verschleiss an Metallteilen, die der Reibabnutzung unterliegen, zu vermindern vermögen. Bekannt ist auch, den Schmierstoffen Antioxidantien zuzugeben, um deren Haltbarkeit und Wirksamkeit zu erhöhen oder über längere Zeiträume zu erhalten.

Bekannt ist auch, z.B. aus der WO 87/02358, dass phosphorhaltige Verbindungen als Schmierölzusätze in Schmierölen für Verbrennungsmotoren, die mit einem katalytischen Abgasreinigungssystem ausgerüstet sind, nicht bedenkenlos angewendet werden können. Es wird vermutet, dass phosphorhaltige Verbindungen den Katalysator desaktivieren (vgl. H.S. Gandhi et al., Applied Catalysis 3, (1982), 79-88).

In WO 87/02358 werden Thiadiazole und deren Salze als Schmierstoffzusätze beschrieben, welche keinen Phosphor enthalten und bei denen deshalb die beschriebenen negativen Aspekte nicht auftreten.

Aus der US 4,659,853 sind Derivate der Isothiocyanate bekannt geworden, ohne dass deren Verwendung näher erläutert worden wäre.

Im Hinblick auf verminderte Brennraumablagerungen werden heute auch erhöhte Anforderungen bezüglich einer Aschearmut der Schmierstoffadditive gestellt.

Aufgabe vorliegender Erfindung ist es, Schmierstoffzusammensetzungen zur Verfügung zu stellen, die Additive enthalten, welche die Metallteile vor extremen Drücken und Reibabnutzung schützen, also einen Verschleissschutz darstellen, und die den Schmierstoff vor oxidativem Abbau schützen, ohne dass das Schmierstoffadditiv beispielsweise schädliche Einflüsse auf ein katalytisches Abgasreinigungssystem ausübt.

Erfindungsgemäss wird das durch eine Zusammensetzung erreicht, enthaltend

a) einen Schmierstoff oder eine Hydraulikflüssigkeit und

b) mindestens eine Verbindung der allgemeinen Formel I

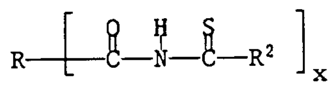 (I),

wobei x = 1 oder 2 ist und,
wenn x = 1 ist,
R die Bedeutung von $R^1$ oder $R^3$-O- hat, oder
wenn x = 2 ist,
R die Bedeutung von $R^8$ hat, und

$R^1$ und $R^3$ Alkyl mit 1 bis 25 C-Atomen, eine unsubstituierte oder $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$c_{12}$-Cycloalkyl-$C_1$-$C_4$-alkylgruppe, Alkenyl mit 2 bis 18 C-Atomen, Phenyl, Naphthyl, $C_7$-$C_{18}$-Aralkyl oder $C_7$-$C_{18}$-Alkaryl bedeuten oder

$R^1$ und $R^3$ Alkyl mit 1 bis 25 C-Atomen, eine unsubstituierte oder $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, Alkenyl mit 2 bis 18 C-Atomen, Phenyl, Naphthyl, $C_7$-$C_{18}$-Aralkyl oder $C_7$-$C_{18}$-Alkaryl, die jeweils mit einer oder mehreren Gruppen der Reihe Halogen, Cyano, Nitro, -$OR^a$ oder -$COOR^b$ substituiert sind, bedeuten, oder

$R^1$ und $R^3$ Alkyl mit 2 bis 25 C-Atomen, eine unsubstituierte oder $C_1$-$C_8$-alkylsubstituierte Cycloalkylgruppe mit 3 bis 10 Ring-C-Atomen, eine unsubstituierte oder $C_1$-$C_8$-alkylsubstituierte $C_3$-$C_{12}$-Cycloalkyl-$C_1$-$C_4$-alkylgruppe, Alkenyl mit 3 bis 18 C-Atomen, $C_7$-$C_{18}$-Aralkyl oder $C_7$-$C_{18}$-Alkaryl, die jeweils durch eine oder mehrere Gruppen der Reihe -O-, -S-, -NH-,

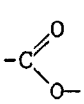 oder 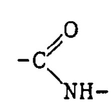

unterbrochen sind, wobei $C_7$-$C_{18}$-Aralkyl oder $C_7$-$C_{18}$-Alkaryl nur in den Alkylresten unterbrochen sind, bedeu-

ten, oder
$R^1$ und $R^3$ Alkyl mit 2 bis 25 C-Atomen, eine unsubstituierte oder $C_1$-$C_8$-alkylsubstituierte Cycloalkylgruppe mit 3 bis 10 Ring-C-Atomen, eine unsubstituierte oder $C_1$-$C_8$-alkylsubstituierte $C_3$-$C_{12}$-Cycloalkyl-$C_1$-$C_4$-alkylgruppe, Alkenyl mit 3 bis 18 C Atomen, $C_7$-$C_{18}$-Aralkyl oder $C_7$-$C_{18}$-Alkaryl, die jeweils durch eine oder mehrere Gruppen der Reihe Halogen, Cyano, Nitro, -$OR^a$ oder -$COOR^b$ substituiert und durch jeweils eine oder mehrere Gruppen der Reihe -O-, -S-, -NH-,

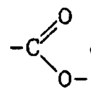 oder 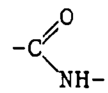

unterbrochen sind, wobei $C_7$-$C_{18}$-Aralkyl oder $C_7$-$C_{18}$-Alkaryl nur in den Alkylresten unterbrochen sind, bedeuten,
wobei $R^a$ die Bedeutung von $R^3$ mit Ausnahme von -$OR^a$ und
$R^b$ die Bedeutung von $R^6$ hat, und
$R^2$ die Bedeutung von -$NR^4R^5$, -$OR^6$ oder -$SR^7$ hat, und
$R^4$ und $R^5$ gleich oder verschieden sind und -H, Alkyl mit 1 bis 23 C-Atomen, eine unsubstituierte oder $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, Phenyl, Naphthyl, $C_7$-$C_{18}$-Aralkyl oder $C_7$-$C_{18}$-Alkaryl bedeuten, oder
$R^4$ und $R^5$, zusammen mit dem sie verbindenden N-Atom, einen Piperidin-, Morpholin-, Hexamethylenimin (Perhydroazepin)-, Pyrrolidin-, Piperazinoder 1-Methylpiperazinrest bilden, und
$R^6$ und $R^7$ Alkyl mit 1 bis 18 C-Atomen bedeuten, oder Alkyl mit 2 bis 20 C-Atomen, das durch eine oder mehrere Gruppen der Reihe -O-, -S-, -NH-,

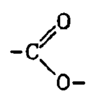 oder 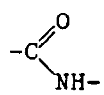

unterbrochen ist, bedeuten, oder eine unsubstituierte oder $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, eine unsubstituierte oder $C_1$-$C_8$-alkylsubstituierte $C_3$-$C_{12}$-Cycloalkyl-$C_1$-$C_4$-alkylgruppe, die durch eine oder mehrere Gruppen der Reihe -O-, -S-, -NH-,

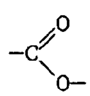 oder 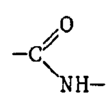

unterbrochen ist, oder Phenyl, Naphthyl, $C_7$-$C_{18}$-Alkaryl oder $C_7$-$C_{18}$-Aralkyl darstellen, und
$R^8$ Alkylen mit 1 bis 18 C-Atomen, Alkylen mit 2 bis 18 C-Atomen, das durch wenigstens eine -O-Gruppe unterbrochen ist, Alkyliden mit 2 bis 20 C-Atomen oder Alkyliden mit 3 bis 20 C-Atomen, das durch wenigstens eine -O-Gruppe unterbrochen ist, darstellt.

$R^1$ und $R^3$ können beispielsweise Alkyl mit 1 bis 25 C-Atomen, eine unsubstituierte oder $C_1$-$C_8$-alkylsubstituierte Cycloalkylgruppe mit 5 bis 12 Ring-C-Atomen, Alkenyl mit 2 bis 18 C-Atomen, Phenyl, Naphthyl, $C_7$-$C_{18}$-Aralkyl oder $C_7$-$C_{18}$-Alkaryl sein, die jeweils mit einer, zwei oder drei, vorzugsweise einer, Gruppe der Reihe Halogen, Cyano, Nitro, -$OR^a$ oder -$COOR^b$ substituiert sind, bedeuten, oder
$R^1$ und $R^3$ können beispielsweise Alkyl mit 2 bis 25 C-Atomen, eine unsubstituierte oder $C_1$-$C_8$-alkylsubstituierte Cycloalkylgruppe mit 5 bis 12 Ring-C-Atomen, Alkenyl mit 3 bis 18 C-Atomen, $C_8$-$C_{18}$-Aralkyl oder $C_8$-$C_{18}$-Alkaryl sein, die jeweils durch eine, zwei oder drei, vorzugsweise eine, Gruppe der Reihe -O-, -S-, -NH-,

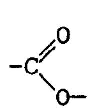 oder 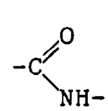

unterbrochen sind, wobei $C_8$-$C_{18}$-Aralkyl oder $C_8$-$C_{18}$-Alkaryl nur in den Alkylresten unterbrochen sind, bedeuten, oder

$R^1$ und $R^3$ können beispielsweise Alkyl mit 2 bis 25 C-Atomen, eine unsubstituierte oder $C_1$-$C_8$-alkylsubstituierte Cycloalkylgruppe mit 5 bis 12 Ring-C-Atomen, Alkenyl mit 3 bis 18 C Atomen, $C_8$-$C_{16}$-Aralkyl oder $C_8$-$C_{18}$-Alkaryl sein, die jeweils durch eine, zwei oder drei, vorzugsweise eine, Gruppe der Reihe Halogen, Cyano, Nitro, -$OR^a$ oder -$COOR^b$ substituiert und jeweils durch eine, zwei oder drei, vorzugsweise eine, Gruppe der Reihe -O-, -S-, -NH-,

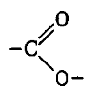 oder 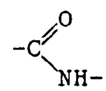

unterbrochen sind, wobei $C_8$-$C_{18}$-Aralkyl oder $C_8$-$C_{18}$-Alkaryl nur in den Alkylresten unterbrochen sind, bedeuten, wobei $R^a$ und Rb die genannten Bedeutungen haben, und

$R^6$ und $R^7$ können beispielsweise Alkyl mit 2 bis 20 C-Atomen, die durch eine, zwei oder drei, vorzugsweise eine, Gruppe der Reihe -O-, -S-, -NH-,

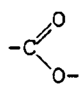 oder 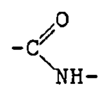

unterbrochen sind, bedeuten.

$R^8$ kann beispielsweise Alkylen mit 1 bis 18 C-Atomen, das durch eine -O-Gruppe unterbrochen ist oder Alkyliden mit 3 bis 20 C-Atomen, das durch eine -O-Gruppe unterbrochen ist, darstellen.

Zweckmässige Zusammensetzungen enthalten mindestens eine Verbindung der allgemeinen Formel I, worin $R^1$ und $R^3$ Alkyl mit 1 bis 18 C-Atomen, Cycloalkyl mit 5 bis 8 Ring-C-Atomen, Phenyl oder $C_7$-$C_{18}$-Aralkyl bedeuten, $R^4$ und $R^5$ gleich oder verschieden sind und -H, Alkyl mit 1 bis 18 C-Atomen, Cycloalkyl mit 5 - 12 Ring-C-Atomen oder Phenyl bedeuten, und $R^6$ und $R^7$ Alkyl mit 1 bis 18 C-Atomen, eine Cycloalkylgruppe mit 5 bis 8 Ring-C-Atomen oder Alkyl mit 2 bis 20 C-Atomen, das durch eine Gruppe

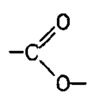

unterbrochen ist oder Phenyl oder $C_7$-$C_{18}$-Aralkyl bedeuten, und $R^8$ Alkylen mit 1 bis 18 C-Atomen oder Alkyliden mit 2 bis 20 C-Atomen darstellt.

Stellt $R^1$ oder $R^3$ einen Alkylrest mit 1 bis 25 C-Atomen dar, so kann die Alkylgruppe geradkettig oder verzweigt sein und kann beispielsweise bedeuten: Methyl, Ethyl, Propyl, Isopropyl, n-Butyl, Isobutyl, t-Butyl, Pentyl, Isopentyl, Hexyl, Heptyl, 3-Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, Icosyl, Henicosyl, Docosyl, 2-Ethylbutyl, 1-Methylpentyl, 1,3-Dimethylbutyl, 1,1,3,3-Tetramethylbutyl, 1-Methylhexyl, Isoheptyl, 1-Methylheptyl, 1,1,3-Trimethylhexyl, 1,1,3,3-Tetramethylhexyl oder 1-Methylundecyl.

Beispiele für $R^6$ und $R^7$ und für zweckmässige Reste $R^1$ und $R^3$ sind Alkylreste mit 1 bis 18 C-Atomen. Beispiele lassen sich sinngemäss obiger Aufstellung entnehmen. Bevorzugt sind Alkylreste wie Ethyl, n-Butyl oder Heptadecyl.

Langkettige Alkylreste können als geradkettige oder verzweigte, als auch gemischtkettige Reste vorliegen, wobei insbesondere auch die verzweigtkettigen Reste als Gemische ihrer Isomeren vorliegen können.

$R^1$ und $R^3$ können auch Alkenyl mit 2 bis 18 C-Atomen bedeuten. Beispiele dafür sind Vinyl, Allyl, 2-Methallyl, Butenyl, wie z.B. 2-Butenyl, Hexenyl, wie z.B. 2-Hexenyl, Decenyl, Undecenyl, wie z.B. 10-Undecenyl, Heptadecenyl oder Oleyl.

Beispiele für die $R^1$, $R^3$, $R^6$ und $R^7$ genannten unsubstituierten Cycloalkylgruppen mit 5 bis 12 C-Atomen sind Cyclopentyl, Cyclohexyl, Cyclooctyl, Cyclododecyl, wobei Cyclohexyl besonders bevorzugt ist. Beispiele für $C_1$-$C_8$-alkylsubstituierte Cycloalkylgrupen mit 5 bis 12 Ring-C-Atomen sind solche, die zweckmässig eine, zwei oder drei Alkylgruppen mit insgesamt 1 bis 8 C-Atomen tragen und es können 2- oder 4-Methylcyclohexyl, Dimethylcyclohexyl, Trimethylcyclohexyl oder t-Butylcyclohexyl genannt werden.

Bedeuten $R^1$, $R^3$, $R^6$ und $R^7$ eine unsubstituierte oder $C_1$-$C_8$-alkylsubstituierte $C_5$-$C_{12}$-Cycloalkyl-$C_1$-$C_4$-

alkylgruppe, so sind damit beispielsweise Reste gemeint, der allgemeinen Formel

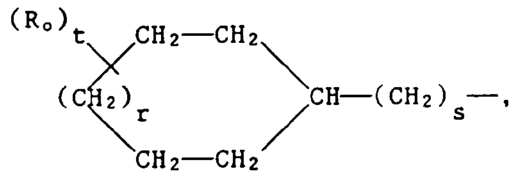

wobei r die Zahlen 0, 1, 2, 3, 4, 5, 6 oder 7, s die Zahlen 1, 2, 3 oder 4 und t die Zahlen 0 oder 1 und grösser, zweckmässig 0, 1, 2 oder 3 bedeuten und $R_o$ Alkyl mit 1 bis 8 C-Atomen, vorzugsweise Methyl, Ethyl, Propyl, n-butyl oder t-Butyl bedeutet.

Zweckmässig sind Reste der Formeln

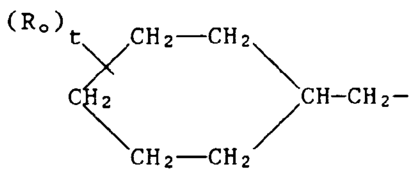

oder

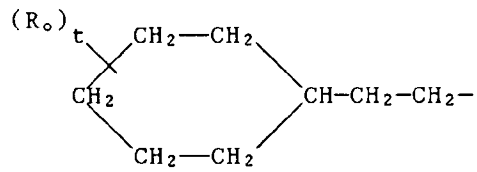

,

wobei t = 0, 1, 2 oder 3 ist und $R_o$ Methyl oder t-Butyl bedeutet und besonders bevorzugt sind für $R_o$ Methyl und für t = 1, 2 oder 3 oder für $R_o$ t-Butyl und t = 1 oder t ist 0.

Die Substituenten $R_1$, $R_3$, $R_6$ und $R_7$ können auch unsubstituierte oder $C_1$-$C_8$-alkylsubstituierte $C_3$-$C_{12}$-Cycloalkylgruppen, die durch eine oder mehrere Gruppen der Reihe -O-, -S-, -HN-,

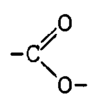

oder

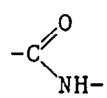

unterbrochen sind, darstellen.

Bevorzugt sind Reste der allgemeinen Formel

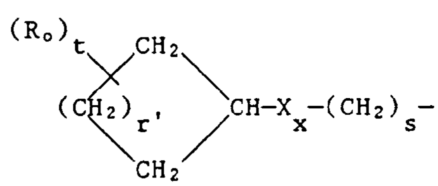

wobei r' die Zahlen 0 oder 1 bis 9, zweckmässig jedoch 3 darstellt. X kann eine der Gruppen der Reihe -O-, -S-, -NH-,

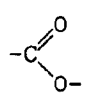

oder

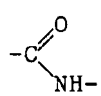

darstellen und bevorzugt ist

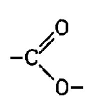

.

s kann die

Bedeutung von 1, 2, 3 oder 4 haben und ist zweckmässig 1. Die Bedeutungen von $R_o$ und t, sowie die Bevorzugungen sind vorstehend angegeben.

Besonders bevorzugt ist eine Unterbrechung in der Alkylgruppe mit

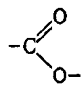 .

Ganz besonders bevorzugt ist eine Gruppe der Formel

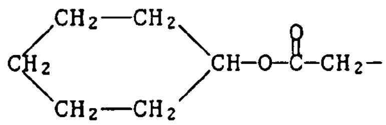 .

Bedeutet $R^1$ oder $R^3$ $C_7$-$C_{18}$-Aralkyl, so können vor allem Naphthyl-$C_1$-$C_8$-Alkyl und insbesondere Phenyl-$C_1$-$C_8$-Alkyl, beispielsweise Benzyl, 2-Phenylethyl (Dihydrostyryl), Methylbenzyl genannt werden.

Zu den Beispielen für $R^1$ und $R^3$, die den Resten $C_7$-$C_{18}$-Alkaryl zugehörig sind, gehören z.B. $C_{11}$-$C_{18}$-Alkylnaphthyl und insbesondere $C_7$-$C_{18}$-Alkylphenyl, vorzugsweise Methylphenyl, Dimethylphenyl, Trimethylphenyl, Ethylphenyl, Isopropylphenyl, t-Butylphenyl, Di-t-butylphenyl oder 2,6-Di-t-butyl-4-methylphenyl.

Der Substituent $R^2$ hat z.B. die Bedeutung von $-NR^4R^5$, wobei $R^4$ und $R^5$ gleich oder verschieden sein können und bevorzugt gleich sind.

Stellen $R^4$ und $R^5$ einen Alkylrest mit 1 bis 23 C-Atomen dar, so können beispielsweise Methyl, Ethyl, n-Propyl, i-Propyl, n-Butyl, i-Butyl, n-Pentyl, n-Hexyl, n-Heptyl, n-Octyl, 2-Ethylhexyl, Nonyl, Decyl, Undecyl, Dodecyl, Heptadecyl oder Henicosyl genannt werden.

Beispiele für $R^2$ als Rest $-NR^4R^5$ sind die N-Butyl-, N-Hexyl-, N-n-Octyl-, N-t-Octyl, N-t-Dodecyl, N-t-Tetradecyl, N-t-Docosyl-, N-Methyl-N-Octyl-, N-Methyl-N-Dodecyl, N-Methyl-N-Hexadecyl-, N-Methyl-N-Octadecyl, N,N-Di-i-Butyl-, N,N-Di-n-Hexyl, N,N-Di-t-Octyl, N,N-Di-2-Ethylhexyl-, N,N-Di-dodecyl-, N-Methyl-N-Phenyl- oder N-Dodecyl-N-Phenylreste.

Im weiteren kann $R^4$ und $R^5$ $C_7$-$C_{18}$-Aralkyl und $C_7$-$C_{18}$-Alkaryl darstellen. Beispiele dafür sind oben aufgeführten Aufzählungen zu entnehmen.

Hat der Substituent $R^2$ die Bedeutung von $-OR^6$ oder $-SR^7$ so sind $R^6$ und $R^7$ beispielsweise Alkyl mit 1 bis 18 C-Atomen. Es sind dazu beispielhaft zu nennen Methyl, Ethyl, Propyl, Isopropyl, n-Butyl, Isobutyl, t-Butyl, Pentyl, Isopentyl, Hexyl, Heptyl, 3-Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, 2-Ethylbutyl, 1-Methylpentyl, 1,3-Dimethylbutyl, 1,1,3,3-Tetramethylbutyl, 1-Methylhexyl, Isoheptyl, 1-Methylheptyl, 1,1,3-Trimethylhexyl, 1,1,3,3-Tetramethylhexyl oder 1-Methylundecyl.

Bevorzugte Beispiele sind Methyl, Ethyl, n-Butyl, n-Octyl, i-Octyl, 2-Ethylhexyl und 1,1,3,3-Tetramethylhexyl.

Zu bevorzugten Beispielen für $R^6$ und $R^7$, welche Alkyl mit 2 bis 20 C-Atomen, die durch eine oder mehrere Gruppen der Reihe -O-, -S-, -NH-,

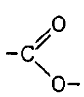 oder 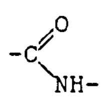

unterbrochen sind, darstellen, gehören Reste der Formel

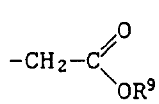 ,

wobei $R^9$ $C_1$-$C_{18}$-Alkyl und vorzugsweise $C_4$-$C_{12}$-Alkyl darstellt.

Bedeuten $R^6$ und $R^7$ beispielsweise $C_7$-$C_{18}$-Alkaryl oder $C_7$-$C_{18}$-Aralkyl, so sind geeignete Beispiele oben

genannter Aufstellung zu entnehmen. Bevorzugt wird ein Benzylrest für $R^6$ und insbesondere für $R^7$.

$R^8$ hat u.a. die Bedeutung von Alkylen mit 1 bis 18 C-Atomen, wozu die Beispiele Methylen, Ethylen, Trimethylen, 2,2-Dimethyl-1,3-propandiyl, Tetramethylen, Pentamethylen, Hexamethylen, Heptamethylen, Octamethylen, Decamethylen oder Dodecamethylen gehören. Trimethylen, Tetramethylen, Hexamethylen und Octamethylen werden bevorzugt.

Beispiele für $R^8$ als Alkylen, das durch 1 oder 2 Sauerstoffatome unterbrochen ist, sind 3-Oxapentan-1,5-diyl; 3,6-Dioxaoctan-1,8-diyl; 2-Oxapropan-1,3-diyl; 2,7-Dioxaoctan-1,8-diyl oder 2,6-Dioxa-4,4-dimethyl-1,7-heptandiyl.

$R^8$ hat auch die Bedeutung von Alkyliden mit 2 bis 20 C-Atomen, zu denen als Beispiele Ethyliden, Propyliden, Butyliden, Pentyliden, 4-Methylpentyliden, Heptyliden, Nonyliden, Tridecyliden, Nonadecyliden, 1-Methylethyliden, 1-Ethylpropyliden genannt werden können.

Beispiele für die Bedeutung von $R^9$ als $C_1$-$C_{18}$-Alkyl und insbesondere $C_4$-$C_{12}$-Alkyl lassen sich sinngemäss obiger Aufzählung der Alkylreste entnehmen.

Ein weiteres Beispiel für eine bevorzugte Gruppe für $R^1$ ist

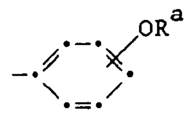 ,

wobei $R^a$ die genannten Bedeutungen hat, und insbesondere bevorzugt für $R^1$ ist

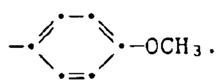

Weitere zweckmässige Zusammensetzungen enthalten eine Verbindung der Formel Ia

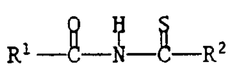 (Ia),

wobei $R^1$ und $R^2$ die oben angegebenen Bedeutungen haben, oder
eine Verbindung der Formel Ib

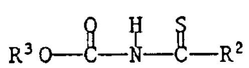 (Ib),

wobei $R^2$ und $R^3$ die oben angegebenen Bedeutungen haben, oder
eine Verbindung der Formel Ic

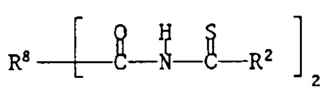 (Ic),

wobei $R^2$ und $R^8$ die oben angegebenen Bedeutungen haben.

Es ist im Umfange vorliegender Erfindung, dass in den Zusammensetzungen auch mehrere Verbindungen der Formel I, respektive Ia, Ib und/oder Ic, in beliebigem Gemisch untereinander, angewendet werden können.

Zu den besonders zweckmässigen Zusammensetzungen sind solche zu zählen, die eine Verbindung der Formel Ia enthalten, wobei

$R^1$ Alkyl mit 1 bis 18 C-Atomen, Phenyl oder Benzyl und $R^2$ -O-($C_1$-$C_{18}$)-Alkyl, -N[($C_1$-$C_{18}$)Alkyl]$_2$, -O-($C_5$-$C_{12}$)-Cycloalkyl, -O-Phenyl, -O-($C_7$-$C_{18}$)-Aralkyl, -S-($C_1$-$C_{12}$)-Alkyl, -S-Phenyl, -S-($C_7$-$C_{18}$)-Aralkyl oder -S-$CH_2$-$COOR^9$, wobei $R^9$ $C_1$-$C_{18}$-Alkyl ist, darstellen.

Eine andere besonders zweckmässige Zusammensetzung enthält eine Verbindung der Formel Ib, wobei $R^2$ -O-($C_1$-$C_{18}$)-Alkyl, -O-($C_5$-$C_{12}$)-Cycloalkyl, -O-Phenyl, -O-($C_7$-$C_{18}$)-Aralkyl, -S-($C_1$-$C_{12}$)-Alkyl, -S-Phenyl, -S-

$(C_7\text{-}C_{18})$-Aralkyl oder $-S\text{-}CH_2\text{-}COOR^9$ darstellt, wobei
$R^9$ $C_1\text{-}C_{18}$-Alkyl ist und
$R^3$ $C_1\text{-}C_{18}$-Alkyl, Cyclohexyl, Phenyl oder Benzyl darstellt.

Eine weitere besonders zweckmässige Zusammensetzung enthält eine Verbindung der Formel Ic, wobei
$R^2$ $-O\text{-}(C_1\text{-}C_{18})$-Alkyl, $-N[(C_4\text{-}C_8)\text{-Alkyl}]_2$, -O-Phenyl, $-O\text{-}(C_7\text{-}C_{18})$-Aralkyl, $-O\text{-}(C_5\text{-}C_{12})$-Cycloalkyl, $-S\text{-}(C_1\text{-}C_{12})$-Alkyl, -S-Phenyl, $-S\text{-}(C_7\text{-}C_{18})$-Aralkyl oder $-S\text{-}CH_2\text{-}COOR^9$ darstellt, wobei
$R^9$ $C_1\text{-}C_{18}$-Alkyl oder $C_5\text{-}C_{12}$-Cycloalkyl ist, und
$R^8$ $C_1\text{-}C_{10}$-Alkylen oder $C_2\text{-}C_{10}$-Alkyliden darstellt.

Zu den bevorzugten Zusammensetzungen sind solche zu zählen, enthaltend eine Verbindung der Formel Ia, wobei
$R^1$ Alkyl mit 9 bis 18 C-Atomen oder Phenyl ist und
$R^2$ $-O\text{-}(C_1\text{-}C_8)$-Alkyl, $-S\text{-}(C_8\text{-}C_{10})$-Alkyl, $-N[(C_4\text{-}C_8\text{-Alkyl}]_2$, $-S\text{-}CH_2\text{-}COO\text{-}(C_2\text{-}C_8)$-Alkyl oder -S-Benzyl darstellt, oder
enthaltend eine Verbindung der Formel Ib wobei
$R^2$ $-O\text{-}(C_2\text{-}C_8)$-Alkyl, $-S\text{-}(C_8\text{-}C_{12})$-Alkyl oder $-N\text{-}[(C_4\text{-}C_8)\text{-Alkyl}]_2$ und
$R^3$ $C_2\text{-}C_4$-Alkyl, Cyclohexyl, oder Phenyl darstellen, oder enthaltend eine Verbindung der Formel Ic, wobei
$R^2$ $-N[(C_4\text{-}C_8)\text{-Alkyl}]_2$ und
$R^8$ $C_3\text{-}C_8$-Alkylen darstellen.

Zusammensetzungen, die besonders bevorzugt werden, enthalten eine Verbindung der Formel Ia, worin
$R^1$ Phenyl und $R^2$ $-SR^7$ oder $-S\text{-}CH_2\text{-}COOR^9$ darstellt und $R^7$ $C_4\text{-}C_{12}$-Alkyl und $R^9$ $C_4\text{-}C_{12}$-Alkyl darstellen, oder enthalten eine Verbindung der Formel Ib, worin
$R^2$ $-SR^7$ oder $-S\text{-}CH_2\text{-}COOR^9$, wobei $R^7$ $C_4\text{-}C_{12}$-Alkyl und $R^9$ $C_4\text{-}C_{12}$-Alkyl sind, und
$R^3$ $C_1\text{-}C_4$-Alkyl, Cyclohexyl oder Phenyl darstellen, oder enthalten eine Verbindung der Formel Ib, wobei
$R^2$ -S-n-Octyl und $R^3$ Ethyl oder $R^2$ -S-( 2-Ethylhexyl) und $R^3$ n-Butyl darstellen.

Die Verbindungen der Formel I sind z.T. aus den nachfolgend zitierten Literaturstellen bekannt oder können beispielsweise nach den an sich bekannten Methoden hergestellt werden.

Solche Methoden werden in H. Hartmann, I. Reuther, Journal für praktische Chemie, Band 315, Heft 1, 1973, S. 144-148; K.H. König, M. Kuge, L. Kaul, M.J. Pletsch, Chemische Berichte 120, Seiten 1251-1253, (1987), und in E. Schröpl, R. Pohloudek-Fabini, Pharmazeutische Zentralhalle, Band 107, (1968), Heft 7, Seiten 493-500 beschrieben.

In Anlehnung an die an sich bekannten Methoden können für die Verbindungen der Formeln Ia und Ib folgende allgemeinen Gleichungen des Herstellungsverfahrens angeführt werden:

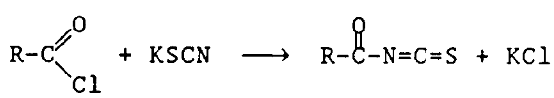

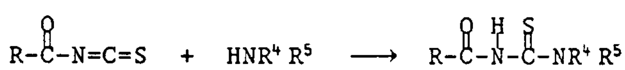

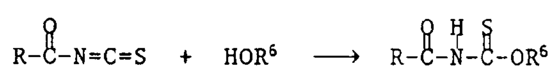

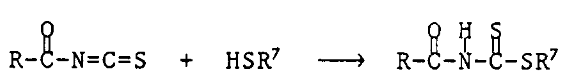

Für Verbindungen der Formel Ic gilt beispielsweise die Gleichung:

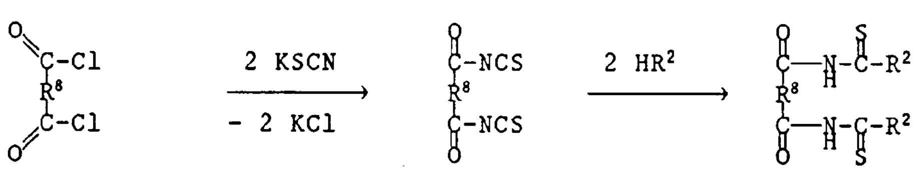

Die Bezeichnungen R, $R^2$, $R^4$, $R^5$, $R^6$, $R^7$ und $R^8$ können die weiter oben erläuterten Bedeutungen haben.

Die Ausgangsstoffe, beispielsweise ein Acylchlorid, ein Thiocyanat und das gewählte Amin, der Alkohol oder das Phenol oder das Mercaptan oder Thiophenol werden beispielsweise in einem polaren aprotischen Lösungsmittel bei Temperaturen zwischen etwa 50°C und 70°C zur Umsetzung gebracht.

Das Thiocyanat kann auch in einem ersten Verfahrensschritt mit einem Halogenameisensäureester umgesetzt werden. Beispiele für solche Ester sind Methylchlorformiat (Chlorameisensäuremethylester), Ethylchlorformiat, Allylchlorformiat, Benzylchlorformiat, Butylchlorformiat, Phenylchlorformiat oder Vinylchlorformiat.

Geeignete Acylchloride lassen sich sinngemäss aus den oben genannten Bedeutungen für R ableiten und dies sind beispielsweise Acetylchlorid, Butyrylchlorid, Oleoylchlorid, 3-Phenyl-propionylchlorid, Benzoylchlorid oder für $R^8$ Glutarsäuredichlorid, Adipinsäuredichlorid, Korksäuredichlorid, Sebacinsäuredichlorid usw.

Als Thiocyanate lassen sich beispielsweise Ammoniumthiocyanat, Natriumthiocyanat und bevorzugt Kaliumthiocyanat aufzählen.

Entsprechend den Bedeutungen für $R^2$ resp. $R^4$, $R^5$, $R^6$ und $R^7$ können als Amine z.B. n-Butylamin, 2-Ethylhexylamin, Dihexylamin, Bis(2-Ethylhexyl)amin, Methylanilin, Piperidin, Morpholin, Hexamethylenimin (Perhydroazepin), Pyrrolidin, 1-Methylpiperazin, Piperazin, als Alkohole oder Phenole beispielhaft Ethanol, Butanol, iso-Butanol, 2-Ethyl-hexanol, Cyclohexanol, Phenol, 4-Methylphenol (p-Kresol) und als Mercaptane oder Thiophenole etwa Octylmercaptan, tert. Dodecylmercaptan, Thioglykolsäureethylester, Thioglycolsäure-2-ethylhexylester oder 3,5-Dimethylthiophenol usw. genannt werden.

Die Aufzählung von Ausgangsprodukten ist als beispielhaft anzusehen. Die vollständigen Ausgangsprodukte ergeben sich sinngemäss und entsprechend aus allen Substituenten, die für R und $R^2$ in Frage kommen.

Die erfindungsgemässen Zusammensetzungen enthalten als weitere Komponente einen Schmierstoff oder eine Hydraulikflüssigkeit. Schmierstoffe sind bevorzugt und es können die an sich bekannten Produkte zur Anwendung gelangen.

Die gesuchten Eigenschaften der erfindungsgemässen Verbindungen kommen auch in den Hydraulikflüssigkeiten voll zum Tragen, wenn auch in diesem Falle der Asche- und Phosphorarmut oder -freiheit nicht die ganz grosse obenerwähnte Bedeutung zukommt.

Die in Frage kommenden Schmierstoffe und Hydraulikflüssigkeiten sind dem Fachmann geläufig und z.B. in Dieter Klamann "Schmierstoffe und verwandte Produkte", Verlag Chemie, Weinheim, 1982, in Schewe-Kobek, "Das Schmiermittel-Taschenbuch", Dr. Alfred Hüthig-Verlag, Heidelberg, 1974, oder in "Ullmanns Encyclopädie der technischen Chemie", Band 13, Seiten 85-94 (Verlag Chemie, Weinheim, 1977) beschrieben.

Beispiele hierfür sind Schmierstoffe und Hydraulikflüssigkeiten auf Basis von Mineralölen, synthetischen Oelen oder Mischungen mineralischer und synthetischer Oele, oder synthetische Schmierstoffe oder Hydraulikflüssigkeiten, beispielsweise solche, die Carbonsäure-Esterderivate darstellen und bei Temperaturen von 200°C und höher verwendet werden können.

Beispiele von synthetischen Schmierstoffen umfassen auch Schmierstoffe auf der Basis eines Diesters einer zweiwertigen Säure mit einem einwertigen Alkohol, wie z.B. Dioctylsebacat oder Dinonyladipat, eines Triesters von Trimethylolpropan mit einer einwertigen Säure oder mit einem Gemisch solcher Säuren, wie z.B. Trimethylolpropantripelargonat, Trimethylolpropan-tricaprylat oder Gemische davon, eines Tetraesters von Pentaerythrit mit einer einwertigen Säure oder mit einem Gemisch solcher Säuren, wie z.B. Pentaerythrit-tetracaprylat, oder eines komplexen Esters von einwertigen und zweiwertigen Säuren mit mehrwertigen Alkoholen, wie z.B. ein komplexer Ester von Trimethylolpropan mit Capryl- und Sebacinsäure oder von einem Gemisch davon.

Besonders geeignet sind neben Mineralölen z.B. Poly-$\alpha$-Olefine, Schmierstoffe auf Esterbasis, Phosphate, Glykole, Polyglykole und Polyalkylenglykole, sowie deren Mischungen mit Wasser.

Die Verbindungen der Formel I sind gut in Schmierstoffen und Hydraulikflüssigkeiten löslich und sind deshalb als Zusätze zu Schmierstoffen und Hydraulikflüssigkeiten besonders geeignet und es ist auf ihre überraschend gute antioxidative und verschleissmindernde Wirkung hinzuweisen.

Beispielsweise in Schmierstoffen für Verbrennungsmotoren, wie z.B. in Verbrennungsmotoren nach dem Otto-Prinzip, vermögen die Verbindungen der Formel I ihre überragenden Eigenschaften zu entfalten. So verhindern oder vermindern die Verbindungen der Formel I in Schmierölen die Reibabnutzung von Metallteilen und wirken im Schmieröl antioxidativ, ohne jedoch nachteilig auf ein katalytisches Abgasreinigungssystem einzuwirken.

Die Verbindungen der Formel I wirken schon in sehr geringen Mengen als Additive in Schmierstoffen und Hydraulikflüssigkeiten. Sie werden den Schmierstoffen und Hydraulikflüssigkeiten zweckmässig in einer Menge von 0,01 bis 5 Gew.-%, vorzugsweise in einer Menge von 0,05 bis 3 Gew.-% und ganz besonders bevorzugt in einer Menge von 0,1 bis 2 Gew.-%, jeweils bezogen auf den Schmierstoff oder die Hydraulikflüssigkeit, beigemischt.

Die erfindungsgemässen Schmierstoffe und Hydraulikflüssigkeiten können zusätzlich andere Additive enthalten, die zugegeben werden, um die Grundeigenschaften von Schmierstoffen und Hydraulikflüssigkeiten noch weiter zu verbessern; dazu gehören: weitere Antioxidantien, Metalldesaktivatoren, Rostinhibitoren, Viskositätsindex-Verbesserer, Stockpunkterniedriger, Dispergiermittel, Detergentien, Tenside, weitere Hochdruck-Zusätze und Antiverschleiss-Additive.

Beispielsweise ist eine Reihe solcher Verbindungen der nachfolgenden Auflistung zu entnehmen.

Beispiele für phenolische Antioxidantien

1. Alkylierte Monophenole

2,6-Di-tert-butylphenol, 2,6-Di-tert-butyl-4-methylphenol, 2,6-Di-tert-butylphenol, 2-tert-Butyl-4,6-dimethylphenol, 2,6-Di-tert-butyl-4-ethylphenol, 2, 6-Di-tert-butyl-4-n-butylphenol, 2, 6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, o-tert-Butylphenol.

2. Alkylierte Hydrochinone

2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol.

3. Hydroxylierte Thiodiphenylether

2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol).

4. Alkyliden-Bisphenole

2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4- oder -5-iso-butylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)-butan, 2, 6-Di-(3-tert-butyl-5-methyl-2-hydroxybenzyl)-4-methylphenol, 1,1,3-Tris-(5-tert-butyl-4-hydroxy-2-methylphenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methylphenyl)-dicyclopentadien, Bis-[2-(3'-tert-butyl-2'-hydroxy-5'-methyl-benzyl)-6-tert-butyl-4-methyl-phenyl]-terephthalat.

5. Benzylverbindungen

1,3,5-Tri-(3,5-di-tert-butyl-4-hydroxybenzyl)-2,4,6-trimethylbenzol, Bis-(3,5-di-tert-butyl-4-hydroxybenzyl)-sulfid, 3,5-Di-tert-butyl-4-hydroxybenzyl-mercaptoessigsäure-isooctylester, Bis-(4-tert-butyl-3-hydroxy-2,6-dimethylbenzyl)-dithiol-terephthalat, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 3,5-Di-tert-butyl-4-hydroxybenzyl-phosphonsäure-dioctadecylester, 3,5-Di-tert-butyl-4-hydroxybenzyl-phosphonsäure-monoethylester, Calcium-salz.

6. Acylaminophenole

4-Hydroxy-laurinsäureanilid, 4-Hydroxy-stearinsäureanilid, 2,4-Bis-octylmercapto-6-(3,5-di-tert-butyl-4-hydroxyanilino)-s-triazin, N-(3,5-di-tert-butyl-4-hydroxyphenyl)-carbaminsäureoctylester.

7. Ester der β-(3,5-Di-tert-butyl-4-hydroxyphenyl)-propionsäure

mit ein- oder mehrwertigen Alkoholen, wie z.B. mit Methanol, Diethylenglycol, Octadecanol, Triethylenglycol, 1,6-Hexandiol, Pentaerythrit, Neopentylglycol, Tris-hydroxyethyl-isocyanurat, Thiodiethylenglycol, Bis-hydroxyethyl-oxalsäurediamid.

8. Ester der β-(5-tert-butyl-4-hydroxy-3-methylphenyl)-propionsäure

mit ein- oder mehrwertigen Alkoholen, wie z.B. mit Methanol, Diethylenglycol, Octadecanol, Triethylenglycol, 1,6-Hexandiol, Pentaerythrit, Neopentylglycol, Tris-hydroxyethyl-isocyanurat, Thiodiethylenglycol, Bis-hydroxyethyl-oxalsäurediamid.

9. Amide der β-(3,5-Di-tert-butyl-4-hydroxyphenyl)-propionsäure,

wie z.B. N,N'-Bis-(3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.

Beispiele für aminische Antioxidantien:

N,N'-Di-isopropyl-p-phenylendiamin, N,N'-Di-sec-butyl-p-phenylendiamin, N,N'-Bis(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-Phenyl-2-naphthylamin, octyliertes Diphenylamin, z.B. p,p'-Di-tert-octyldiphenylamin, 4-n-Butylaminophenol, 4-Butyrylamino-phenol, 4-Nonanoylamino-phenol, 4-Dodecanoylamino-phenol, 4-Octadecanoylamino-phenol, Di-(4-methoxyphenyl)-amin, 2,6-Di-tert-butyl-4-dimethylamino-methyl-phenol, 2,4'-Diamino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl-/tert-Octyldiphenylaminen, 2,3-Dihydro-3,3-dimethyl-4H-1,4-benzothiazin, Phenothiazin, N-Allylphenothiazin.

Beispiele für weitere Antioxidantien:

Aliphatische oder aromatische Phosphite, Ester der Thiodipropionsäure oder der Thiodiessigsäure, oder Salze der Dithiocarbamid- oder Dithiophosphorsäure.

Beispiele für Metall-Desaktivatoren, z.B. für Kupfer, sind:

Triazole, Benztriazole und deren Derivate, Tolutriazole und deren Derivate, 2-Mercaptobenzthiazol, 2-Mercaptobenztriazol, 2,5-Dimercaptobenztriazol, 2,5-Dimercaptobenzthiadiazol, 5,5'-Methylenbisbenztriazol, 4,5,6,7-Tetrahydrobenztriazol, Salicyliden-propylendiamin, Salicylaminoguanidin und dessen Salze, sowie Diethanolaminomethyltolyltriazol, Di(2-ethylhexyl)aminomethyltolyltriazol.

Beispiele für Rost-Inhibitoren sind:

a) Organische Säuren, ihre Ester, Metallsalze und Anhydride, z.B.:
N-Oleoyl-sarcosin, Sorbitan-mono-oleat, Blei-naphthenat, Alkenylbernsteinsäureanhydrid, z.B. Dodecenylbernsteinsäure-anhydrid, Alkenylbernsteinsäure-Teilester und -Teilamide, 4-Nonylphenoxy-essigsäure.
b) Stickstoffhaltige Verbindungen, z.B.:
I. Primäre, sekundäre oder tertiäre aliphatische oder cycloaliphatische Amine und Amin-Salze von organischen und anorganischen Säuren, z.B. öllösliche Alkylammoniumcarboxylate.
II. Heterocyclische Verbindungen, z.B.:
Substituierte Imidazoline und Oxazoline.
c) Phosphorhaltige Verbindungen, z.B.:
Aminsalze von Phosphorsäurepartialestern oder Phosphonsäurepartialestern, Zinkdialkyldithiophosphate.
d) Schwefelhaltige Verbindungen, z.B.:
Barium-dinonylnaphthalin-sulfonate, Calciumpetroleum-sulfonate.

Beispiele für Viskositätsindex-Verbesserer sind:

Polyacrylate, Polymethacrylate, Vinylpyrrolidon/Methacrylat-Copolymere, Polyvinylpyrrolidone, Polybutene, Olefin-Copolymere, Styrol/Acrylat-Copolymere, Polyether.

Beispiele für Stockpunkterniedriger sind:

Polymethacrylat, alkylierte Naphthalinderivate.

Beispiele für Dispergiermittel/Tenside sind:

Polybutenylbernsteinsäureamide oder -imide, Polybutenylphosphonsäurederivate, basische Magnesium-, Calcium-, und Bariumsulfonate und -phenolate.

Beispiele für Verschleissschutz-Additive und Hochdruckzusätze sind:

Schwefel und/oder Phosphor und/oder Halogen enthaltende Verbindungen, wie geschwefelte pflanzliche Oele, Zinkdialkyldithiophosphate, Tritolylphosphat, chlorierte Paraffine, Alkyl- und Aryldi- und tri-sulfide, Triphenylphosphorothionate.

Ein Teil der erfindungsgemäss einsetzbaren Verbindungen sind neu. Die vorliegende Erfindung betrifft daher auch neue Verbindungen der allgemeinen Formel II

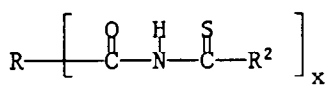

(II),

wobei x = 1 oder 2 ist,
$R^2$ die Bedeutung von $-OR^6$ oder $-SR^7$ hat, und,
wenn x = 1 ist,
R die Bedeutung von $R^1$ oder $R^3$-O- hat,
und $R^1$ Alkyl mit 1 bis 25 C-Atomen, eine unsubstituierte oder $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, Alkenyl mit 2 bis 18 C-Atomen, Phenyl, Naphthyl, $C_7$-$C_{18}$-Aralkyl oder $C_7$-$C_{18}$-Alkaryl bedeutet, oder
$R^3$ Alkyl mit 9 bis 25 C-Atomen, eine unsubstituierte oder $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, Alkenyl mit 5 bis 18 C-Atomen, $C_7$-$C_{18}$-Aralkyl oder $C_7$-$C_{18}$-Alkaryl bedeutet, oder
$R^1$ und $R^3$ Alkyl mit 1 bis 25 C-Atomen, eine unsubstituierte oder $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, Alkenyl mit 2 bis 18 C-Atomen, Phenyl, Naphthyl, $C_7$-$C_{18}$-Aralkyl oder $C_7$-$C_{18}$-Alkaryl, die jeweils mit einer oder mehreren Gruppen der Reihe Halogen, Cyano, Nitro, $-OR^a$ oder $-COOR^b$ substituiert sind, bedeuten, oder
$R^1$ und $R^3$ Alkyl mit 2 bis 25 C-Atomen, eine unsubstituierte oder $C_1$-$C_8$-alkylsubstituierte Cycloalkylgruppe mit 3 bis 10 Ring-C-Atomen, eine unsubstituierte oder $C_1$-$C_8$-alkylsubstituierte $C_3$-$C_{12}$-Cycloalkyl-$C_1$-$C_4$-alkylgruppe, Alkenyl mit 3 bis 18 C-Atomen, $C_7$-$C_{18}$-Aralkyl oder $C_7$-$C_{18}$-Alkaryl, die jeweils durch eine oder mehrere Gruppen der Reihe -O-, -S-, -NH-,

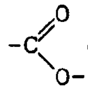 oder 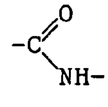

unterbrochen sind, wobei $C_7$-$C_{18}$-Aralkyl oder $C_7$-$C_{18}$-Alkaryl nur in den Alkylresten unterbrochen sind, bedeuten, oder
$R^1$ und $R^3$ Alkyl mit 2 bis 25 C-Atomen, eine unsubstituierte oder $C_1$-$C_8$-alkylsubstituierte Cycloalkylgruppe mit 3 bis 10 Ring-C-Atomen, eine unsubstituierte oder $C_1$-$C_8$-alkylsubstituierte $C_3$-$C_{12}$-Cycloalkyl-$C_1$-$C_4$-alkylgruppe, Alkenyl mit 3 bis 18 C-Atomen, $C_7$-$C_{18}$-Aralkyl oder $C_7$-$C_{18}$-Alkaryl, die jeweils durch eine oder mehrere Gruppen der Reihe Halogen, Cyano, Nitro, $-OR^a$ oder $-COOR^b$ substituiert und durch jeweils eine oder mehrere

Gruppen der Reihe -O-, -S-, -NH-,

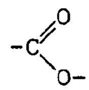

oder

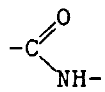

unterbrochen sind, wobei $C_7$-$C_{18}$-Aralkyl oder $C_7$-$C_{18}$-Alkaryl nur in den Alkylresten unterbrochen sind, bedeuten,
wobei $R^a$ die Bedeutung von $R^3$ mit Ausnahme von -$OR^a$ und
$R^b$ die Bedeutung von Alkyl mit 1 bis 18 C-Atomen oder von Alkyl mit 2 bis 20 C-Atomen, die durch eine oder mehrere Gruppen der Reihe -O-, -S-, -NH-,

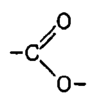 oder 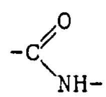

unterbrochen sind, oder einer unsubstituierten oder $C_1$-$C_8$-alkylsubstituierten Cycloalkylgruppe mit 5 bis 12 Ring-C-Atomen oder von Phenyl, von Naphthyl, von $C_7$-$C_{18}$-Alkaryl oder von $C_7$-$C_{18}$-Aralkyl hat, und $R^6$ und $R^7$ Alkyl mit 11 bis 18 C-Atomen oder Alkyl mit 2 bis 20 C-Atomen, das durch eine oder mehrere Gruppen der Reihe -O-, -S-, -NH-,

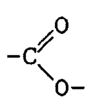 oder 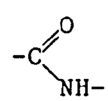

unterbrochen ist, oder eine unsubstituierte oder eine $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, eine unsubstituierte oder $C_1$-$C_8$-alkylsubstituierte $C_3$-$C_{12}$-Cycloalkyl-$C_1$-$C_4$-alkylgruppe, die durch eine oder mehrere Gruppen der Reihe -O-, -S-, -NH-,

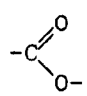 oder 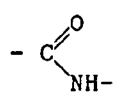

unterbrochen ist,
$C_7$-$C_{18}$-Alkaryl oder $C_7$-$C_{18}$-Aralkyl darstellen, oder,
wenn x = 2 ist,
R die Bedeutung von $R^8$ hat und
$R^8$ Alkylen mit 1 bis 18 C-Atomen, Alkylen mit 2 bis 18 C-Atomen, das durch wenigstens eine -O-Gruppe unterbrochen ist, Alkyliden mit 2 bis 20 C-Atomen oder Alkyliden mit 3 bis 20 C-Atomen, das durch wenigstens eine -O-Gruppe unterbrochen ist, darstellt.

In vorstehenden Ausdeutungen bedeuten die Ausdrücke "eine oder mehrere Gruppen" respektive "durch wenigstens eine Gruppe" beispielsweise eine, zwei oder drei Gruppen und bevorzugt wird eine Gruppe.

Hat $R^3$ die Bedeutung von Alkyl, so sind Alkylgruppen mit 10 bis 25 C-Atomen zweckmässig, Alkylgruppen mit 12 bis 25 C-Atomen bevorzugt und Alkylgruppen mit 15 bis 18 C-Atomen besonders bevorzugt.

Hat $R^3$ die Bedeutung von Alkenyl, so sind Alkenylgruppen mit 8 bis 18 C-Atomen zweckmässig und Alkenylgruppen mit 12 bis 18 C-Atomen bevorzugt.

Bedeutet $R^6$ Alkyl, so sind Alkylgruppen mit 12 bis 18 C-Atomen zweckmässig und Alkylgruppen mit 15

bis 18 C-Atomen bevorzugt.

Zweckmässige Verbindungen haben die Formel IIa

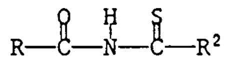

(IIa),

worin R die Bedeutung von $R^1$ oder $R^3$-O- hat und $R^1$, $R^2$ und $R^3$ die oben angegebenen Bedeutungen haben.

Weitere zweckmässige Verbindungen weisen die Formel IIc auf,

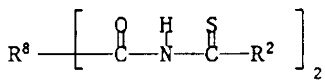

(IIc),

worin $R^2$ und $R^8$ die oben angegebenen Bedeutungen haben.

Bevorzugte Verbindungen haben die Formel IIa, worin R die Bedeutung von $R^1$ oder -O-$R^3$ hat und $R^1$ Phenyl oder Alkyl mit 1 bis 18 C-Atomen und $R^3$ Alkyl mit 1 bis 18 C-Atomen oder Phenyl bedeutet und $R^2$ die Bedeutung von -$SR^7$ hat.

In besonders bevorzugten Verbindungen der Formel IIa hat $R^2$ die Bedeutung von -$SR^7$, wobei $R^7$ die Bedeutung von Alkyl mit 1 bis 18 C-Atomen,

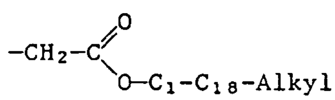

oder Phenyl-$C_1$-$C_4$-Alkyl hat.

Besonders bevorzugte Verbindungen weisen die Formeln

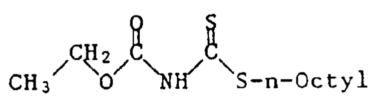

oder

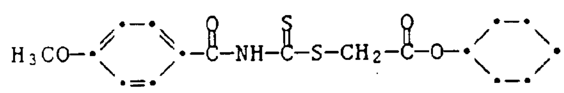

auf.

Vorliegende Erfindung betrifft auch die Verwendung der Verbindungen der Formeln I und II als Verschleissschutzadditive, beispielsweise in Schmierstoffen, für Metallteile die einer Reibabnutzung unterliegen und als Antioxidantien in Schmierstoffen.

Anhand der nachfolgenden Beispiele ist die Erfindung näher erläutert. Alle Angaben in Prozenten oder Teilen beziehen sich auf das Gewicht, sofern nicht anders angegeben.

Beispiel 1:

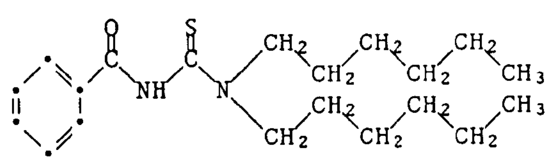

Zu einer Lösung von 9,9 g (0,1 mol) Kaliumthiocyanat in 100 ml Aceton werden bei Rückflusstemperatur innert 15 min. 14,35 g (0,1 mol) Benzoylchlorid zugetropft. Nach weiteren 30 min. Rühren bei Rückfluss werden zur nunmehr weissen Suspension 18,9 g (0,1 mol) Dihexylamin bei Rückfluss innert 20 min. zugetropft. Nach weiteren 5 Std. Rückfluss wird die Reaktionsmischung auf 400 ml 10%ige Salzsäure gegossen und mit Toluol und Ethylacetat extrahiert. Die organische Phase wird mit Magnesiumsulfat getrocknet und eingedampft. Das Rohprodukt wird durch wenig Silicagel eluiert (Toluol: Ethylacetat 4:1). Nach Entfernung des Lösungsmittels

erhält man 22,3 g eines braun-orangen Oels (64 % d.Th.).

Beispiel 12:

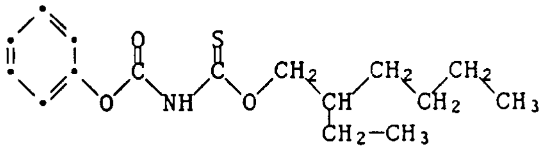

Zu einer übersättigten Lösung von 6,9 g (0,07 mol) Kaliumthiocyanat in 80 ml Ethylacetat werden bei 50° innert 30 min. 11,1 g (0,07 mol) Chlorameisensäurephenylester zugetropft. Die gelbe Suspension wird noch 90 min. bei 60° gerührt. Danach werden bei gleicher Temperatur 9,2 g (0,07 mol) 2-Ethyl-1-hexanol zugetropft. Bei 60-65° wird 20 Std. weitergerührt. Dann werden 40 ml Wasser zugegeben und die auf Raumtemperatur abgekühlte Emulsion wird im Scheidetrichter getrennt. Die organische Phase wird über Magnesiumsulfat getrocknet und eingedampft. Das Rohprodukt wird über eine kurze Silicagel-Säule wie vorbeschrieben eluiert: Ausbeute 12,3 g (57 % d.Th.).

Analog den Beispielen 1 und 12 werden die Beispiele 2 bis 11 und 13 bis 26 gemäss Tabelle ausgeführt. Die gewählte Methode ist jeweils näher bezeichnet.

| Bsp. Nr. | Verbindung | Methode gemäss Bsp. | Ausbeute (% d. Theorie) | Aspekt | $^1$H-NMR-Absorption der Gruppe CONHCS 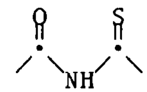 [$CDCl_3$, ppm] | Analyse [berechnet / gefunden] [%] C | H | N | S |
|---|---|---|---|---|---|---|---|---|---|
| 1 | 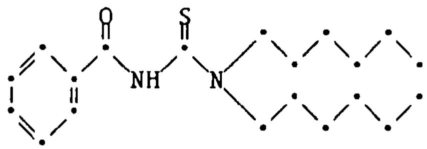 | | 64 | rot-braunes Oel | 8,5 | 68,92<br>69,27 | 9,25<br>9,29 | 8,04<br>7,77 | 13,77<br>13,66 |
| 2 | 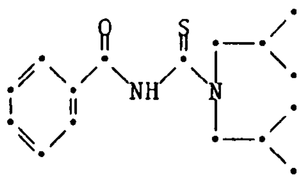 | 1 | 96 | orange-braunes Pulver Smp. 124-126 | 8,4 | 65,71<br>65,75 | 8,27<br>8,25 | 9,58<br>9,47 | 10,96<br>10,95 |
| 3 | 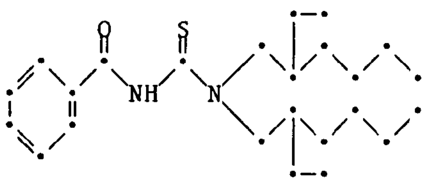 | 12 | 81 | orange-braunes Oel | 8,4 | 71,24<br>71,47 | 9,96<br>9,80 | 6,92<br>6,82 | 7,92<br>7,98 |
| 4 | 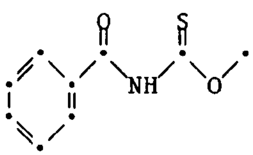 | 1 | | weisses Pulver Smp. 94 | 9,2 | 55,37<br>55,72 | 4,65<br>4,81 | 7,17<br>6,97 | 16,42<br>16,14 |

| Bsp. Nr. | Verbindung | Methode gemäss Bsp. | Ausbeute (% d. Theorie) | Aspekt | $^1$H-NMR-Absorption der Gruppe CONHCS 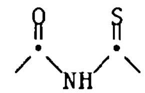 [$CDCl_3$, ppm] | Analyse [berechnet / gefunden] [%] C | H | N | S |
|---|---|---|---|---|---|---|---|---|---|
| 5 | 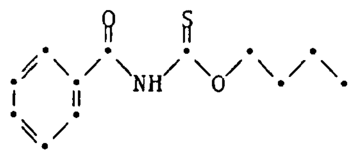 | 1 | 61 | gelbes Pulver Smp. 46-48 | 9,25 | 60,73<br>61,18 | 6,37<br>6,49 | 5,90<br>5,73 | 13,51<br>13,46 |
| 6 | 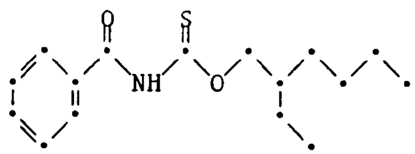 | 1 | 76 | gelbes Oel | 9,2 | 65,49<br>65,60 | 7,90<br>7,95 | 4,77<br>4,97 | 10,93<br>11,00 |
| 7 | 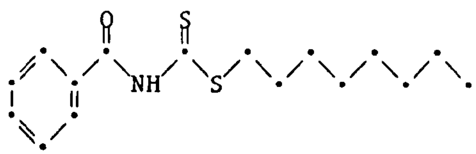 | 1 | 93 | gelbes Harz | 10,1 | 62,10<br>62,35 | 7,49<br>7,62 | 4,53<br>4,51 | 20,72<br>20,55 |
| 8 | 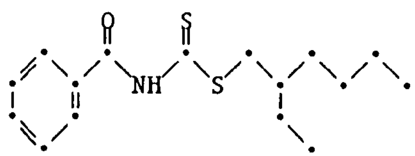 | 1 | 67 | orange-gelbes Oel | 10,05 | 62,10<br>61,97 | 7,49<br>7,60 | 4,53<br>4,55 | 20,72<br>20,61 |

| Bsp. Nr. | Verbindung | Methode gemäss Bsp. | Ausbeute (% d. Theorie) | Aspekt | $^1$H-NMR-Absorption der Gruppe CONHCS 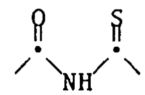 [$CDCl_3$, ppm] | Analyse [berechnet / gefunden] [%] C | H | N | S |
|---|---|---|---|---|---|---|---|---|---|
| 9 | 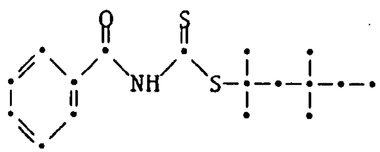 | 1 | 48 | oranges Oel | 9,6 | 63,12<br>63,04 | 7,79<br>7,68 | 4,33<br>4,34 | 19,82<br>19,32 |
| 10 | 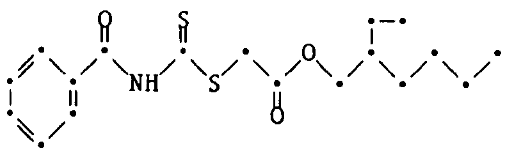 | 1 | 62 | gelbes Pulver Smp. 72-74 | 10,1 | 59,81<br>59,86 | 7,13<br>7,16 | 3,67<br>3,71 | 16,81<br>16,59 |
| 11 | 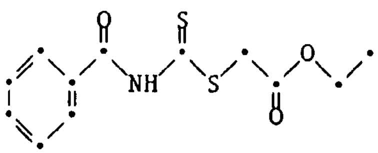 | 1 | 53 | gelbes Pulver Smp. 124-125 | 10,2 | 50,87<br>50,83 | 4,62<br>4,63 | 4,94<br>4,90 | 22,63<br>22,83 |
| 12 | 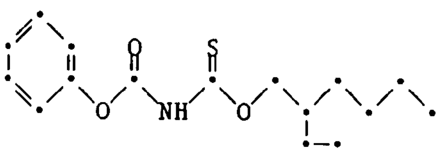 | | 57 | gelbes Oel | 8,5 | 62,11<br>62,36 | 7,49<br>7,63 | 4,53<br>4,46 | 10,36<br>10,25 |

| Bsp. Nr. | Verbindung | Methode gemäss Bsp. | Ausbeute (% d. Theorie) | Aspekt | $^1$ H-NMR-Absorption der Gruppe CONHCS 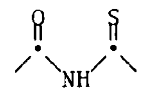 [$CDCl_3$, ppm] | Analyse [berechnet / gefunden] [%] C | H | N | S |
|---|---|---|---|---|---|---|---|---|---|
| 13 | 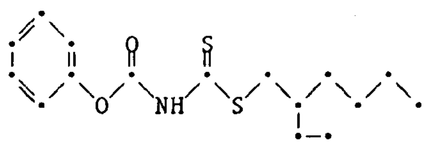 | 12 | 21 | rötliches Oel | 9,3 | | | | |
| 14 | 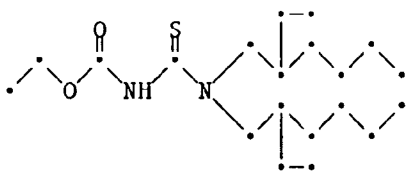 | 1 | 37 | gelbes Oel | 7,2 | 64,47<br>65,94 | 10,82<br>11,14 | 7,52<br>7,63 | 8,60<br>8,19 |
| 15 | 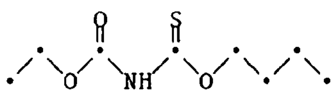 | 1 | 65 | gelbes Oel | 8,5 | 46,81<br>47,00 | 7,37<br>7,52 | 6,82<br>6,79 | 15,62<br>15,67 |
| 16 | 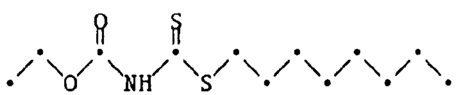 | 12 | 84 | gelbes Harz | 9,2 | 51,95<br>53,01 | 8,36<br>8,44 | 5,05<br>4,85 | 23,11<br>22,39 |
| 17 | 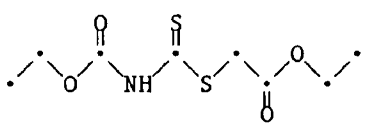 | 1 | 62 | oranges Pulver Smp. 89-90 | 9,1 | 38,23<br>38,15 | 5,21<br>5,13 | 5,57<br>5,97 | 25,51<br>25,61 |

| Bsp. Nr. | Verbindung | Methode gemäss Bsp. | Ausbeute (% d. Theorie) | Aspekt | $^{1}$H-NMR-Absorption der Gruppe CONHCS 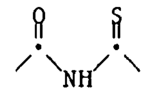 [$CDCl_3$, ppm] | Analyse C | [berechnet / gefunden] H | N | [%] S |
|---|---|---|---|---|---|---|---|---|---|
| 18 | 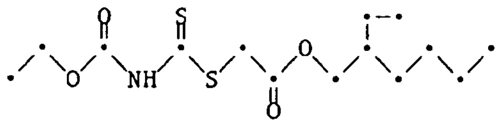 | 1 | 62 | gelbes Oel | 9,1 | 50,12<br>49,75 | 7,51<br>7,48 | 4,18<br>4,23 | 19,11<br>19,02 |
| 19 | 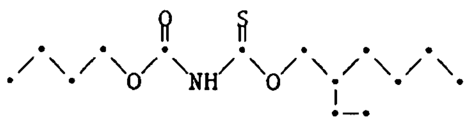 | 12 | 88 | gelbliches Oel | 8,3 | 58,10<br>58,18 | 9,40<br>9,33 | 4,84<br>4,77 | 11,08<br>11,15 |
| 20 | 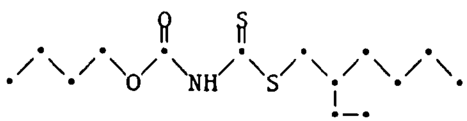 | 12 | 74 | gelbes Oel | 9,1 | 55,04<br>55,13 | 8,91<br>8,96 | 4,59<br>4,54 | 20,99<br>20,93 |
| 21 | 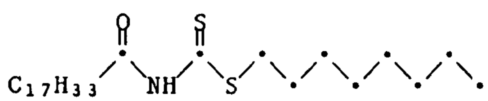 | 12 | 70 | gelbe Flüssigkeit | 9,55 | 69,02<br>69,27 | 10,94<br>10,85 | 2,98<br>2,82 | 13,65<br>13,34 |
| 22 | 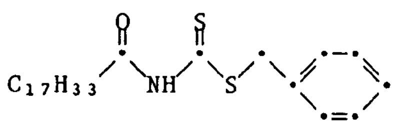 | 12 | 31 | gelbes Oel | 9,5 | 69,75<br>69,81 | 9,23<br>9,38 | 3,13<br>3,00 | 14,32<br>13,78 |

| Bsp. Nr. | Verbindung | Methode gemäss Bsp. | Ausbeute (% d. Theorie) | Aspekt | 1 H-NMR-Absorption der Gruppe CONHCS 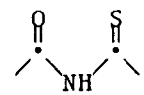 [$CDCl_3$, ppm] | Analyse [berechnet / gefunden] [%] C | H | N | S |
|---|---|---|---|---|---|---|---|---|---|
| 23 | $(CH_2)_3$ 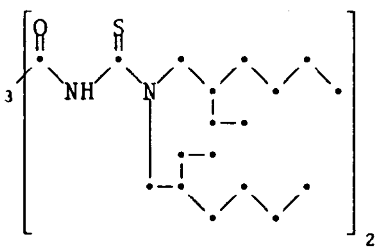 | 1 | 65 | gelbes viskoses Oel | 9,5 breit | 67,19<br>67,24 | 10,99<br>10,83 | 8,04<br>7,92 | 9,20<br>9,43 |
| 24 | $(CH_2)_4$ 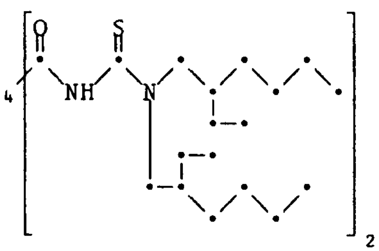 | 1 | 75 | gelbes Harz | 8,45 | 67,55<br>67,54 | 11,05<br>11,27 | 7,88<br>7,73 | 9,02<br>9,06 |

| Bsp. Nr. | Verbindung | Methode gemäss Bsp. | Ausbeute (% d. Theorie) | Aspekt | $^1$ H-NMR-Absorption der Gruppe CONHCS 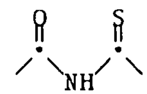 [$CDCl_3$, ppm] | Analyse C | [berechnet / gefunden] H | N | [%] S |
|---|---|---|---|---|---|---|---|---|---|
| 25 | 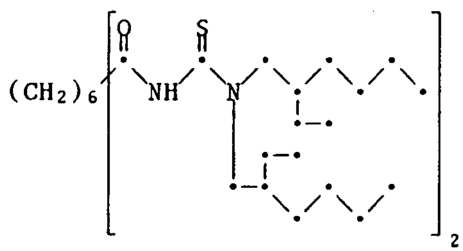 | 1 | 79 | gelbes Harz | 8,75 | 68,24<br>68,42 | 11,18<br>10,83 | 7,58<br>7,56 | 8,67<br>8,70 |
| 26 | 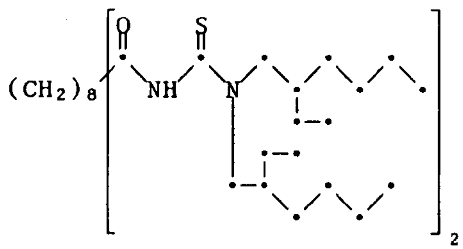 | 1 | 88 | oranges Harz | 8,4 | 68,87<br>69,19 | 11,30<br>11,20 | 7,30<br>7,22 | 8,36<br>8,38 |

Beispiel 27: Test auf Verschleissschutz

Zur Prüfung auf Eignung als Verschleissschutzadditiv wird die ASTM-Standardmethode D-2783-81 unter Verwendung des Shell-Vierkugelapparates herangezogen. Als Basisöl wird Catenex® P941 der Fa. Shell verwendet, dem die in der Tabelle angegebene Menge an Verbindung gemäss dem jeweils genannten Beispiel zugegeben wird. Ermittelt werden

a) Die Schweisslast WL (Weld Load) als die Last (in kg), bei der die 4 Kugeln innerhalb von 10 sec. zusammenschweissen, und

b) der mittlere Verschleiss-Narben-Durchmesser (Wear Scar Diameter) bei einer Last von 40 kg während 1 Std. (in mm).

| Verbindung aus Beispiel | Zusatzmenge (Gew.-%) | Weld Load (kg) | Wear Scar Diameter (mm) |
|---|---|---|---|
| 1 | 0,25<br>1 | -<br>1800 | 0,49<br>0,50 |
| 2 | 0,25<br>1 | -<br>1800 | 0,51<br>0,51 |
| 3 | 0,25<br>1 | -<br>1800 | 0,55<br>0,50 |
| 4 | 0,25<br>1<br>2,5 | -<br>2000<br>2400 | 0,53<br>0,58<br>- |
| 5 | 0,25<br>1<br>2,5 | -<br>2200<br>2400 | 0,53<br>0,53<br>- |
| 6 | 0,25<br>1<br>2,5 | -<br>2000<br>2400 | 0,53<br>0,51<br>- |
| 7 | 0,25<br>1,0<br>2,5 | -<br>2000<br>2200 | 0,52<br>0,56<br>- |
| 8 | 0,25<br>1,0<br>2,5 | -<br>2000<br>2200 | 0,50<br>0,55<br>- |
| 9 | 0,25<br>1,0<br>2,5 | -<br>2200<br>2400 | 0,52<br>0,50<br>- |
| 10 | 0,25<br>1,0 | -<br>1800 | 0,53<br>0,56 |
| 11 | 0,25<br>1,0 | -<br>2200 | 0,57<br>0,59 |
| 12 | 0,25<br>1,0<br>2,5 | -<br>2000<br>2000 | 0,48<br>0,49<br>- |
| 13 | 0,25<br>1,0<br>2,5 | -<br>2000<br>2200 | 0,49<br>0,54<br>- |

| Verbindung aus Beispiel | Zusatzmenge (Gew.-%) | Weld Load (kg) | Wear Scar Diameter (mm) |
|---|---|---|---|
| 14 | 0,25<br>1,0 | <br>1800 | 0,55<br>0,57 |
| 15 | 0,25<br>1,0<br>2,5 | -<br>2200<br>2400 | 0,51<br>0,58<br>- |
| 16 | 0,25<br>1,0<br>2,5 | -<br>2000<br>2400 | 0,56<br>0,63<br>- |
| 17 | 0,25<br>1,0 | -<br>2200 | 0,62<br>- |
| 18 | 0,25<br>1,0<br>2,5 | -<br>2200<br>2800 | 0,58<br>0,65<br>- |
| 19 | 0,25<br>1,0<br>2,5 | -<br>2000<br>2000 | 0,53<br>0,57<br>- |
| 20 | 0,25<br>1,0<br>2,5 | -<br>2000<br>2400 | 0,54<br>0,61<br>- |
| 21 | 0,25<br>1,0 | -<br>1800 | 0,67<br>0,45 |
| 22 | 0,25<br>1,0<br>2,5 | -<br>2000<br>2400 | 0,45<br>0,50<br>- |
| 23 | 0,25<br>1,0 | -<br>1800 | 1,51<br>0,52 |
| 24 | 0,25<br>1,0 | -<br>1800 | 0,53<br>0,54 |
| 25 | 0,25<br>1,0 | -<br>1800 | 0,52<br>0,53 |
| 26 | 0,25<br>1,0 | -<br>1800 | 0,51<br>0,54 |
| Referenz | ohne Additiv | 1400 | 0,70 |

Beispiel 28: Test auf Stabilisierung gegen oxidativen Abbau (TFOUT: Thin Film Oxygen Uptake Test)

Dieser Test ist eine modifizierte Form des Rotary Bomb Test für Mineralöle (ASTM D 2272). Eine detaillierte Beschreibung findet sich bei C.S.Ku, S.M.Hsu, Lubrication Engineering 40, (1984), Seiten 75-83. Das Testöl ist in diesem Versuch ein kommerzielles Motorenöl 15W40, mit ca. der Hälfte des üblichen Gehaltes an

Zinkdithiophosphates (0,75 % ZnDTP, 550 ppm P, 1160 ppm Zn). Das zu testende Additiv wird im Oel in Gegenwart von Wasser (2 %), einer oxidierten/nitrierten Benzinfraktion (4 %) und eines Gemisches von flüssigen Metallnaphthenaten (4 %) bei 610 kPa Sauerstoffdruck und 160°C auf seine stabilisierende Wirkung getestet. Das Wasser und die beiden flüssigen Katalysatoren für den Test werden unter der Bezeichnung Standard Reference Material 1817 vom National Bureau of Standards (NBS) bezogen, mit Bescheinigung für die Analyse. Der Test ist abgeschlossen, wenn im Druck/Zeit-Diagramm ein deutlicher Knick die einsetzende Oxidation am Ende der Induktionsperiode (min.) anzeigt.

Eine lange Induktionsperiode bedeutet eine gute stabilisierende Wirkung des Additives.

| Verbindung aus Beispiel | Zusatzmenge (Gew. %) | Induktionsperiode (min.) |
|---|---|---|
| 5 | 0,5 | 107 |
| 6 | 0,5 | 109 |
| 7 | 0,5 | 123 |
| 9 | 0,5 | 126 |
| 15 | 0,5 | 117 |
| 16 | 0,5 | 129 |
| Referenz | ohne Additiv | 83 |

## Patentansprüche

### Patentansprüche für folgende Vertragsstaaten : BE, DE, FR, GB, IT, NL

1. Zusammensetzung enthaltend
   a) einen Schmierstoff oder eine Hydraulikflüssigkeit und
   b) mindestens eine Verbindung der allgemeinen Formel I

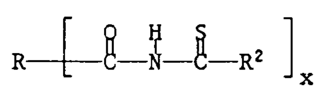

(I),

wobei x = 1 oder 2 ist und,
wenn x = 1 ist,
R die Bedeutung von $R^1$ oder $R^3$-O- hat, oder
wenn x = 2 ist,
R die Bedeutung von $R^8$ hat, und
$R^1$ und $R^3$ Alkyl mit 1 bis 25 C-Atomen, eine unsubstituierte oder $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, Alkenyl mit 2 bis 18 C-Atomen, Phenyl, Naphthyl, $C_7$-$C_{18}$-Aralkyl oder $C_7$-$C_{18}$-Alkaryl bedeuten oder
$R^1$ und $R^3$ Alkyl mit 1 bis 25 C-Atomen, eine unsubstituierte oder $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, Alkenyl mit 2 bis 18 C-Atomen, Phenyl, Naphthyl, $C_7$-$C_{18}$-Aralkyl oder $C_7$-$C_{18}$-Alkaryl, die jeweils mit einer oder mehreren Gruppen der Reihe Halogen, Cyano, Nitro, -$OR^a$ oder -$COOR^b$ substituiert sind, bedeuten, oder
$R^1$ und $R^3$ Alkyl mit 2 bis 25 C-Atomen, eine unsubstituierte oder $C_1$-$C_8$-alkylsubstituierte Cycloalkylgruppe mit 3 bis 10 Ring-C-Atomen, eine unsubstituierte oder $C_1$-$C_8$-alkylsubstituierte $C_3$-$C_{12}$-Cycloalkyl-$C_1$-

$C_4$-alkylgruppe, Alkenyl mit 3 bis 18 C-Atomen, $C_7$-$C_{18}$-Aralkyl oder $C_7$-$C_{18}$-Alkaryl, die jeweils durch eine oder mehrere Gruppen der Reihe -O-, -S-, -NH-,

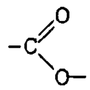 oder 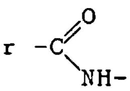

unterbrochen sind, wobei $C_7$-$C_{18}$-Aralkyl oder $C_7$-$C_{18}$-Alkaryl nur in den Alkylresten unterbrochen sind, bedeuten, oder
$R^1$ und $R^3$ Alkyl mit 2 bis 25 C-Atomen, eine unsubstituierte oder $C_1$-$C_8$-alkylsubstituierte Cycloalkylgruppe mit 3 bis 10 Ring-C-Atomen, eine unsubstituierte oder $C_1$-$C_8$-alkylsubstituierte $C_3$-$C_{12}$-Cycloalkyl-$C_1$-$C_4$-alkylgruppe, Alkenyl mit 3 bis 18 C Atomen, $C_7$-$C_{18}$-Aralkyl oder $C_7$-$C_{18}$-Alkaryl, die jeweils durch eine oder mehrere Gruppen der Reihe Halogen, Cyano, Nitro, -$OR^a$ oder -$COOR^b$ substituiert und durch jeweils eine oder mehrere Gruppen der Reihe -O-, -S-, -NH-,

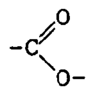 oder 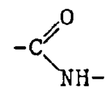

unterbrochen sind, wobei $C_7$-$C_{18}$-Aralkyl oder $C_7$-$C_{18}$-Alkaryl nur in den Alkylresten unterbrochen sind, bedeuten,
wobei $R^a$ die Bedeutung von $R^3$ mit Ausnahme von -$OR^a$ und
$R^b$ die Bedeutung von $R^6$ hat, und
$R^2$ die Bedeutung von -$NR^4$ $R^5$, -$OR^6$ oder -$SR^7$ hat, und
$R^4$ und $R^5$ gleich oder verschieden sind und -H, Alkyl mit 1 bis 23 C-Atomen, eine unsubstituierte oder $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, Phenyl, Naphthyl, $C_7$-$C_{18}$-Aralkyl oder $C_7$-$C_{18}$-Alkaryl bedeuten, oder
$R^4$ und $R^5$, zusammen mit dem sie verbindenden N-Atom, einen Piperidin-, Morpholin-, Hexamethylenimin (Perhydroazepin)-, Pyrrolidin-, Piperazin- oder 1-Methylpiperazinrest bilden, und
$R^6$ und $R^7$ Alkyl mit 1 bis 18 C-Atomen bedeuten, oder Alkyl mit 2 bis 20 C-Atomen, die durch eine oder mehrere Gruppen der Reihe -O-, -S-, -NH-,

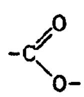 oder 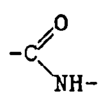

unterbrochen sind, bedeuten, oder eine unsubstituierte oder $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, eine unsubstituierte oder $C_1$-$C_8$-alkylsubstituierte $C_3$-$C_{12}$-Cycloalkyl-$C_1$-$C_4$-alkylgruppe, die durch eine oder mehrere Gruppen der Reihe -O-, -S-, -NH-,

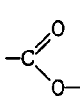 oder 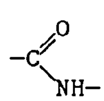

unterbrochen ist, oder Phenyl, Naphthyl, $C_7$-$C_{18}$-Alkaryl oder $C_7$-$C_{18}$-Aralkyl darstellen, und
$R^8$ Alkylen mit 1 bis 18 C-Atomen, Alkylen mit 2 bis 18 C-Atomen, das durch wenigstens eine -O-Gruppe unterbrochen ist, Alkyliden mit 2 bis 20 C-Atomen oder Alkyliden mit 3 bis 20 C-Atomen, das durch wenigstens eine -O-Gruppe unterbrochen ist, darstellt.

2. Zusammensetzungen nach Anspruch 1 enthaltend mindestens eine Verbindung der allgemeinen Formel I, worin $R^1$ und $R^3$ Alkyl mit 1 bis 18 C-Atomen, Cycloalkyl mit 5 bis 8 Ring-C-Atomen, Phenyl oder $C_7$-$C_{18}$-Aralkyl bedeuten, $R^4$ und $R^5$ gleich oder verschieden sind und -H, Alkyl mit 1 bis 18 C-Atomen, Cycloalkyl mit 5 bis 12 Ring-C-Atomen oder Phenyl bedeuten, und $R^6$ und $R^7$ Alkyl mit 1 bis 18 C-Atomen, eine Cycloalkylgruppe mit 5 bis 8 Ring-C-Atomen oder Alkyl mit 2 bis 20 C-Atomen, das durch eine Gruppe

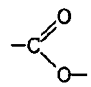

unterbrochen ist oder Phenyl oder $C_7$-$C_{18}$-Aralkyl bedeuten, und $R^8$ Alkylen mit 1 bis 18 C-Atomen oder Alkyliden mit 2 bis 20 C-Atomen darstellt.

3. Zusammensetzung nach Anspruch 1, enthaltend eine Verbindung der Formel Ia

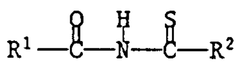

(Ia).

4. Zusammensetzung nach Anspruch 1, enthaltend eine Verbindung der Formel Ib

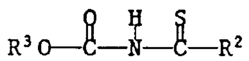

(Ib).

5. Zusammensetzung nach Anspruch 1, enthaltend eine Verbindung der Formel Ic

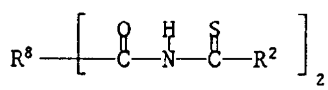

(Ic).

6. Zusammensetzung nach Anspruch 3, enthaltend eine Verbindung der Formel Ia, wobei $R^1$ Alkyl mit 1 bis 18 C-Atomen, Phenyl oder Benzyl und $R^2$ -O-($C_1$-$C_{18}$)-Alkyl, -N[($C_1$-$C_{18}$)Alkyl]$_2$, -O-($C_5$-$C_{12}$)-Cycloalkyl, -O-Phenyl, -O-($C_7$-$C_{18}$)-Aralkyl, -S-($C_1$-$C_{12}$)-Alkyl, -S-Phenyl, -S-($C_7$-$C_{18}$)-Aralkyl oder -S-$CH_2$-COOR$^9$, wobei $R^9$ $C_1$-$C_{18}$-Alkyl ist, darstellen.

7. Zusammensetzung nach Anspruch 3, enthaltend eine Verbindung der Formel Ia, wobei $R^1$ Alkyl mit 9 bis 18 C-Atomen oder Phenyl ist und $R^2$ -O-($C_1$-$C_{18}$)-Alkyl, -S-($C_8$-$C_{10}$)-Alkyl, N[($C_4$-$C_8$)-Alkyl]$_2$, -S-$CH_2$-COO($C_2$-$C_8$)-Alkyl oder -S-Benzyl darstellt.

8. Zusammensetzung nach Anspruch 4, enthaltend eine Verbindung der Formel Ib, wobei $R^2$ -O-($C_1$-$C_{18}$)-Alkyl, -O-($C_5$-$C_{12}$)-Cycloalkyl, -O-Phenyl, -O-($C_7$-$C_{18}$)-Aralkyl, -S-($C_1$-$C_{12}$)-Alkyl, -S-Phenyl, -S-($C_7$-$C_{18}$)-Aralkyl oder -S-$CH_2$-COOR$^9$, wobei
$R^9$ $C_1$-$C_{18}$-Alkyl ist, und
$R^3$ $C_1$-$C_{18}$-Alkyl, Cyclohexyl, Phenyl oder Benzyl darstellen.

9. Zusammensetzung nach Anspruch 4, enthaltend eine Verbindung der Formel Ib, wobei $R^2$ -O-($C_2$-$C_8$)Alkyl, S-($C_8$-$C_{12}$)-Alkyl oder -N-[($C_4$-$C_8$)-Alkyl]$_2$ und
$R^3$ $C_2$-$C_4$-Alkyl, Cyclohexyl oder Phenyl darstellen.

10. Zusammensetzung nach Anspruch 5, enthaltend eine Verbindung der Formel Ic, wobei $R^2$ -O-($C_1$-$C_{18}$)-Alkyl, -N[($C_4$-$C_8$)-Alkyl]$_2$, -O-Phenyl, -O-($C_7$-$C_{18}$)-Aralkyl, -O-($C_5$-$C_{12}$)Cycloalkyl, -S-($C_1$-$C_{12}$)-Alkyl, -S-Phenyl, -S-($C_7$-$C_{18}$)-Aralkyl oder -S-$CH_2$-COOR$^9$ darstellt, wobei
$R^9$ $C_1$-$C_{18}$-Alkyl und
$R^8$ $C_1$-$C_{10}$-Alkylen oder $C_2$-$C_{10}$-Alkyliden darstellen.

11. Zusammensetzung nach Anspruch 5, enthaltend eine Verbindung der Formel Ic, wobei
$R^2$ -N[($C_4$-$C_8$)-Alkyl]$_2$ und
$R^8$ $C_3$-$C_8$-Alkylen darstellen.

12. Zusammensetzung nach Anspruch 3, enthaltend eine Verbindung der Formel Ia, worin $R^1$ Phenyl und $R^2$ $SR^7$ oder $-S-CH_2COOR^9$ darstellt und $R^7$ $C_4$-$C_{12}$-Alkyl und $R^9$ $C_4$-$C_{12}$-Alkyl darstellen.

13. Zusammensetzung nach Anspruch 4, enthaltend eine Verbindung der Formel Ib, worin $R^2$ $-SR^7$ oder $-S-CH_2-COOR^9$, wobei $R^7$ $C_4$-$C_{12}$-Alkyl und $R^9$ $C_4$-$C_{12}$-Alkyl sind, und $R^3$ $C_1$-$C_4$-Alkyl, Cyclohexyl oder Phenyl darstellen.

14. Zusammensetzung nach Anspruch 4, enthaltend eine Verbindung der Formel Ib, wobei $R^2$ -S-n-Octyl und $R^3$ Ethyl darstellen und/oder eine Verbindung der Formel Ib, worin $R^2$ -S-(2-Ethylhexyl) und $R^3$ n-Butyl darstellen.

15. Verbindungen der allgemeinen Formel II

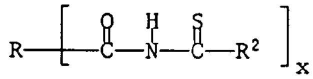

(II),

wobei x = 1 oder 2 ist,
$R^2$ die Bedeutung von $-OR^6$ oder $-SR^7$ hat, und,
wenn x = 1 ist,
R die Bedeutung von $R^1$ oder $R^3$-O- hat,
und $R^1$ Alkyl mit 1 bis 25 C-Atomen, eine unsubstituierte oder $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, Alkenyl mit 2 bis 18 C-Atomen, Phenyl, Naphthyl, $C_7$-$C_{18}$-Aralkyl oder $C_7$-$C_{18}$-Alkaryl bedeutet, oder
$R^3$ Alkyl mit 9 bis 25 C-Atomen, eine unsubstituierte oder $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, Alkenyl mit 5 bis 18 C-Atomen, $C_7$-$C_{18}$-Aralkyl oder $C_7$-$C_{18}$- Alkaryl bedeutet, oder
$R^1$ und $R^3$ Alkyl mit 1 bis 25 C-Atomen, eine unsubstituierte oder $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, Alkenyl mit 2 bis 18 C-Atomen, Phenyl, Naphthyl, $C_7$-$C_{18}$-Aralkyl oder $C_7$-$C_{18}$-Alkaryl, die jeweils mit einer oder mehreren Gruppen der Reihe Halogen, Cyano, Nitro, $-OR^a$ oder $-COOR^b$ substituiert sind, bedeuten, oder
$R^1$ und $R^3$ Alkyl mit 2 bis 25 C-Atomen, eine unsubstituierte oder $C_1$-$C_8$-alkylsubstituierte Cycloalkylgruppe mit 3 bis 10 Ring-C-Atomen, eine unsubstituierte oder $C_1$-$C_8$-alkylsubstituierte $C_3$-$C_{12}$-Cycloalkyl-$C_1$-$C_4$-alkylgruppe, Alkenyl mit 3 bis 18 C-Atomen, $C_7$-$C_{18}$-Aralkyl oder $C_7$-$C_{18}$-Alkaryl, die jeweils durch eine oder mehrere Gruppen der Reihe -O-, -S-, -NH-,

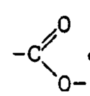 oder 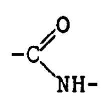

unterbrochen sind, wobei $C_7$-$C_{18}$-Aralkyl oder $C_7$-$C_{18}$-Alkaryl nur in den Alkylresten unterbrochen sind, bedeuten, oder
$R^1$ und $R^3$ Alkyl mit 2 bis 25 C-Atomen, eine unsubstituierte oder $C_1$-$C_8$-alkylsubstituierte Cycloalkylgruppe mit 3 bis 10 Ring-C-Atomen, eine unsubstituierte oder $C_1$-$C_8$-alkylsubstituierte $C_3$-$C_{12}$-Cycloalkyl-$C_1$-$C_4$-alkylgruppe, Alkenyl mit 3 bis 18 C-Atomen, $C_7$-$C_{18}$-Aralkyl oder $C_7$-$C_{18}$-Alkaryl, die jeweils durch eine oder mehrere Gruppen der Reihe Halogen, Cyano, Nitro, $-OR^a$ oder $-COOR^b$ substituiert und durch jeweils eine oder mehrere Gruppen der Reihe -O-, -S-, -NH-,

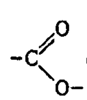 oder 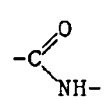

unterbrochen sind, wobei $C_7$-$C_{18}$-Aralkyl oder $C_7$-$C_{18}$-Alkaryl nur in den Alkylresten unterbrochen sind, bedeuten, wobei $R^a$ die Bedeutung von $R^3$ mit Ausnahme von $-OR^a$ und $R^b$ die Bedeutung von Alkyl mit 1 bis 18 C-Atomen oder von Alkyl mit 2 bis 20 C-Atomen, die durch eine oder mehrere Gruppen der Reihe -O-, -S-, -NH-,

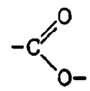 oder 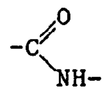

unterbrochen sind, oder einer unsubstituierten oder $C_1$-$C_8$-alkylsubstituierten Cycloalkylgruppe mit 5 bis 12 Ring-C-Atomen oder von Phenyl, von Naphthyl, von $C_7$-$C_{18}$-Alkaryl oder von $C_7$-$C_{18}$-Aralkyl hat, und $R^6$ und $R^7$ Alkyl mit 11 bis 18 C-Atomen oder Alkyl mit 2 bis 20 C-Atomen, das durch eine oder mehrere Gruppen der Reihe -O-, -S-, -NH-,

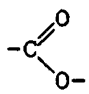 oder 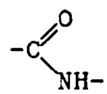

unterbrochen ist, oder eine unsubstituierte oder eine $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, eine unsubstituierte oder $C_1$-$C_8$-alkylsubstituierte $C_3$-$C_{12}$-Cycloalkyl-$C_1$-$C_4$-alkylgruppe, die durch eine oder mehrere Gruppen der Reihe -O-, -S-, -NH-,

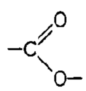 oder 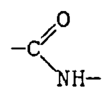

unterbrochen ist,
$C_7$-$C_{18}$-Alkaryl oder $C_7$-$C_{18}$-Aralkyl darstellen, oder,
wenn x = 2 ist,
R die Bedeutung von $R^8$ hat und
$R^8$ Alkylen mit 1 bis 18 C-Atomen, Alkylen mit 2 bis 18 C-Atomen, das durch wenigstens eine -O-Gruppe unterbrochen ist, Alkyliden mit 2 bis 20 C-Atomen oder Alkyliden mit 3 bis 20 C-Atomen, das durch wenigstens eine -O-Gruppe unterbrochen ist, darstellt.

**16.** Verbindungen gemäss Anspruch 15 der Formel IIa

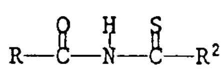 (IIa).

**17.** Verbindungen gemäss Anspruch 15 der Formel IIc

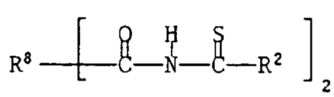 (IIc).

**18.** Verbindungen gemäss Anspruch 16, worin R die Bedeutung von $R^1$ oder $-O-R^3$ hat und $R^1$ Phenyl oder Alkyl mit 1 bis 18 C-Atomen und $R^3$ Alkyl mit 10 bis 18 C-Atomen oder Phenyl bedeutet und $R^2$ die Bedeutung von $-SR^7$ hat.

**19.** Verbindungen gemäss Anspruch 18, worin $R^7$ die Bedeutung von Alkyl mit 12 bis 18 C-Atomen, $-CH_2$-$COO$-$C_1$-$C_{18}$-Alkyl oder Phenyl-$C_1$-$C_4$-Alkyl hat.

20. Verwendung der Verbindungen der Formel I gemäss Anspruch 1 und der Formel II gemäss Anspruch 15 als Hochdruck- und Verschleissschutzadditive und als Antioxidantien in Schmierstoffen und Hydraulikflüssigkeiten.

**Patentansprüche für folgenden Vertragsstaat : ES**

1. Zusammensetzung enthaltend
   a) einen Schmierstoff oder eine Hydraulikflüssigkeit und
   b) mindestens eine Verbindung der allgemeinen Formel I

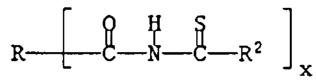

(I),

wobei x = 1 oder 2 ist und,
wenn x = 1 ist,
R die Bedeutung von $R^1$ oder $R^3$-O- hat, oder
wenn x = 2 ist,
R die Bedeutung von $R^8$ hat, und
$R^1$ und $R^3$ Alkyl mit 1 bis 25 C-Atomen, eine unsubstituierte oder $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, Alkenyl mit 2 bis 18 C-Atomen, Phenyl, Naphthyl, $C_7$-$C_{18}$-Aralkyl oder $C_7$-$C_{18}$-Alkaryl bedeuten oder
$R^1$ und $R^3$ Alkyl mit 1 bis 25 C-Atomen, eine unsubstituierte oder $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, Alkenyl mit 2 bis 18 C-Atomen, Phenyl, Naphthyl, $C_7$-$C_{18}$-Aralkyl oder $C_7$-$C_{18}$-Alkaryl, die jeweils mit einer oder mehreren Gruppen der Reihe Halogen, Cyano, Nitro, -$OR^a$ oder -$COOR^b$ substituiert sind, bedeuten, oder
$R^1$ und $R^3$ Alkyl mit 2 bis 25 C-Atomen, eine unsubstituierte oder $C_1$-$C_8$-alkylsubstituierte Cycloalkylgruppe mit 3 bis 10 Ring-C-Atomen, eine unsubstituierte oder $C_1$-$C_8$-alkylsubstituierte $C_3$-$C_{12}$-Cycloalkyl-$C_1$-$C_4$-alkylgruppe, Alkenyl mit 3 bis 18 C-Atomen, $C_7$-$C_{18}$-Aralkyl oder $C_7$-$C_{18}$-Alkaryl, die jeweils durch eine oder mehrere Gruppen der Reihe -O-, -S-, -NH-,

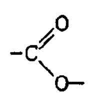 oder 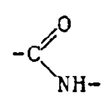

unterbrochen sind, wobei $C_7$-$C_{18}$-Aralkyl oder $C_7$-$C_{18}$-Alkaryl nur in den Alkylresten unterbrochen sind, bedeuten, oder
$R^1$ und $R^3$ Alkyl mit 2 bis 25 C-Atomen, eine unsubstituierte oder $C_1$-$C_8$-alkylsubstituierte Cycloalkylgruppe mit 3 bis 10 Ring-C-Atomen, eine unsubstituierte oder $C_1$-$C_8$-alkylsubstituierte $C_3$-$C_{12}$-Cycloalkyl-$C_1$-$C_4$-alkylgruppe, Alkenyl mit 3 bis 18 C Atomen, $C_7$-$C_{18}$-Aralkyl oder $C_7$-$C_{18}$-Alkaryl, die jeweils durch eine oder mehrere Gruppen der Reihe Halogen, Cyano, Nitro, -$OR^a$ oder -$COOR^b$ substituiert und durch jeweils eine oder mehrere Gruppen der Reihe -O-, -S-, -NH-,

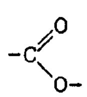 oder 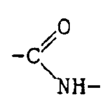

unterbrochen sind, wobei $C_7$-$C_{18}$-Aralkyl oder $C_7$-$C_{18}$-Alkaryl nur in den Alkylresten unterbrochen sind, bedeuten,
wobei $R^a$ die Bedeutung von $R^3$ mit Ausnahme von -$OR^a$ und
$R^b$ die Bedeutung von $R^6$ hat, und
$R^2$ die Bedeutung von -$NR^4$ $R^5$, -$OR^6$ oder -$SR^7$ hat, und
$R^4$ und $R^5$ gleich oder verschieden sind und -H, Alkyl mit 1 bis 23 C-Atomen, eine unsubstituierte oder

$C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, Phenyl, Naphthyl, $C_7$-$C_{18}$-Aralkyl oder $C_7$-$C_{18}$-Alkaryl bedeuten, oder

$R^4$ und $R^5$, zusammen mit dem sie verbindenden N-Atom, einen Piperidin-, Morpholin-, Hexamethylenimin (Perhydroazepin)-, Pyrrolidin-, Piperazinoder 1-Methylpiperazinrest bilden, und

$R^6$ und $R^7$ Alkyl mit 1 bis 18 C-Atomen bedeuten, oder Alkyl mit 2 bis 20 C-Atomen, die durch eine oder mehrere Gruppen der Reihe -O-, -S-, -NH-,

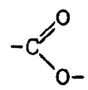 oder 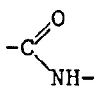

unterbrochen sind, bedeuten, oder eine unsubstituierte oder $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, eine unsubstituierte oder $C_1$-$C_8$-alkylsubstituierte $C_3$-$C_{12}$-Cycloalkyl-$C_1$-$C_4$-alkylgruppe, die durch eine oder mehrere Gruppen der Reihe -O-, -S-, -NH-,

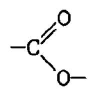 oder 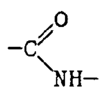

unterbrochen ist, oder Phenyl, Naphthyl, $C_7$-$C_{18}$-Alkaryl oder $C_7$-$C_{18}$-Aralkyl darstellen, und

$R^8$ Alkylen mit 1 bis 18 C-Atomen, Alkylen mit 2 bis 18 C-Atomen, das durch wenigstens eine -O-Gruppe unterbrochen ist, Alkyliden mit 2 bis 20 C-Atomen oder Alkyliden mit 3 bis 20 C-Atomen, das durch wenigstens eine -O-Gruppe unterbrochen ist, darstellt.

2. Zusammensetzungen nach Anspruch 1 enthaltend mindestens eine Verbindung der allgemeinen Formel I, worin $R^1$ und $R^3$ Alkyl mit 1 bis 18 C-Atomen, Cycloalkyl mit 5 bis 8 Ring-C-Atomen, Phenyl oder $C_7$-$C_{18}$-Aralkyl bedeuten, $R^4$ und $R^5$ gleich oder verschieden sind und -H, Alkyl mit 1 bis 18 C-Atomen, Cycloalkyl mit 5 bis 12 Ring-C-Atomen oder Phenyl bedeuten, und $R^6$ und $R^7$ Alkyl mit 1 bis 18 C-Atomen, eine Cycloalkylgruppe mit 5 bis 8 Ring-C-Atomen oder Alkyl mit 2 bis 20 C-Atomen, das durch eine Gruppe

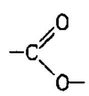

unterbrochen ist oder Phenyl oder $C_7$-$C_{18}$-Aralkyl bedeuten, und $R^8$ Alkylen mit 1 bis 18 C-Atomen oder Alkyliden mit 2 bis 20 C-Atomen darstellt.

3. Zusammensetzung nach Anspruch 1, enthaltend eine Verbindung der Formel Ia

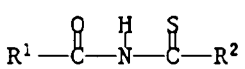 (Ia).

4. Zusammensetzung nach Anspruch 1, enthaltend eine Verbindung der Formel Ib

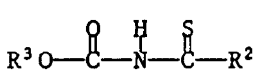 (Ib).

5. Zusammensetzung nach Anspruch 1, enthaltend eine Verbindung der Formel Ic

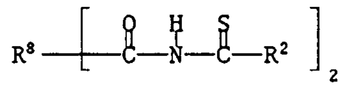 (Ic).

6. Zusammensetzung nach Anspruch 3, enthaltend eine Verbindung der Formel Ia, wobei $R^1$ Alkyl mit 1 bis 18 C-Atomen, Phenyl oder Benzyl und $R^2$ -O-($C_1$-$C_{18}$)-Alkyl, -N[($C_1$-$C_{18}$)Alkyl]$_2$, -O-($C_5$-$C_{12}$)-Cycloalkyl, -O-Phenyl, -O-($C_7$-$C_{18}$)-Aralkyl, -S-($C_1$-$C_{12}$)-Alkyl, -S-Phenyl, -S-($C_7$-$C_{18}$)-Aralkyl oder -S-$CH_2$-$COOR^9$, wobei $R^9$ $C_1$-$C_{18}$-Alkyl ist, darstellen.

7. Zusammensetzung nach Anspruch 3, enthaltend eine Verbindung der Formel Ia, wobei $R^1$ Alkyl mit 9 bis 18 C-Atomen oder Phenyl ist und $R^2$ -O-($C_1$-$C_{18}$)-Alkyl, -S-($C_8$-$C_{10}$)-Alkyl, -N[($C_4$-$C_8$)-Alkyl]$_2$, -S-$CH_2$-COO($C_2$-$C_8$)-Alkyl oder -S-Benzyl darstellt.

8. Zusammensetzung nach Anspruch 4, enthaltend eine Verbindung der Formel Ib, wobei $R^2$ -O-($C_1$-$C_{18}$)-Alkyl, -O-($C_5$-$C_{12}$)-Cycloalkyl, -O-Phenyl, -O-($C_7$-$C_{18}$)-Aralkyl, -S-($C_1$-$C_{12}$)-Alkyl, -S-Phenyl, -S-($C_7$-$C_{18}$)-Aralkyl oder -S-$CH_2$-$COOR^9$, wobei
$R^9$ $C_1$-$C_{18}$-Alkyl ist, und
$R^3$ $C_1$-$C_{18}$-Alkyl, Cyclohexyl, Phenyl oder Benzyl darstellen.

9. Zusammensetzung nach Anspruch 4, enthaltend eine Verbindung der Formel Ib, wobei $R^2$ -O-($C_2$-$C_8$)-Alkyl, -S-($C_8$-$C_{12}$)-Alkyl oder -N-[($C_4$-$C_8$)-Alkyl]$_2$ und
$R^3$ $C_2$-$C_4$-Alkyl, Cyclohexyl oder Phenyl darstellen.

10. Zusammensetzung nach Anspruch 5, enthaltend eine Verbindung der Formel Ic, wobei $R^2$ -O-($C_1$-$C_{18}$)-Alkyl, -N[($C_4$-$C_8$)-Alkyl]$_2$, -O-Phenyl, -O-($C_7$-$C_{18}$)-Aralkyl, -O-($C_5$-$C_{12}$)Cycloalkyl, -S-($C_1$-$C_{12}$)-Alkyl, -S-Phenyl, -S-($C_7$-$C_{18}$)-Aralkyl oder -S-$CH_2$-$COOR^9$ darstellt, wobei
$R^9$ $C_1$-$C_{18}$-Alkyl und
$R^8$ $C_1$-$C_{10}$-Alkylen oder $C_2$-$C_{10}$-Alkyliden darstellen.

11. Zusammensetzung nach Anspruch 5, enthaltend eine Verbindung der Formel Ic, wobei
$R^2$ -N[($C_4$-$C_8$)Alkyl]$_2$ und
$R^8$ $C_3$-$C_8$-Alkylen darstellen.

12. Zusammensetzung nach Anspruch 3, enthaltend eine Verbindung der Formel Ia, worin $R^1$ Phenyl und $R^2$ $SR^7$ oder -S-$CH_2COOR^9$ darstellt und $R^7$ $C_4$-$C_{12}$-Alkyl und $R^9$ $C_4$-$C_{12}$-Alkyl darstellen.

13. Zusammensetzung nach Anspruch 4, enthaltend eine Verbindung der Formel Ib, worin
$R^2$ -$SR^7$ oder -S-$CH_2$-$COOR^9$, wobei $R^7$ $C_4$-$C_{12}$-Alkyl und $R^9$ $C_4$-$C_{12}$-Alkyl sind, und $R^3$ $C_1$-$C_4$-Alkyl, Cyclohexyl oder Phenyl darstellen.

14. Zusammensetzung nach Anspruch 4, enthaltend eine Verbindung der Formel Ib, wobei $R^2$ -S-n-Octyl und $R^3$ Ethyl darstellen und/oder eine Verbindung der Formel Ib, worin $R^2$ -S-(2-Ethylhexyl) und $R^3$ n-Butyl darstellen.

15. Verfahren zur Herstellung von Verbindungen der allgemeinen Formel II

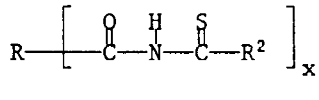 (II),

wobei x = 1 oder 2 ist,
$R^2$ die Bedeutung von -$OR^6$ oder -$SR^7$ hat, und,
wenn x = 1 ist,
R die Bedeutung von $R^1$ oder $R^3$-O- hat,
und $R^1$ Alkyl mit 1 bis 25 C-Atomen, eine unsubstituierte oder
$C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, Alkenyl mit 2 bis 18 C-Atomen, Phenyl, Naphthyl,

$C_7$-$C_{18}$-Aralkyl oder $C_7$-$C_{18}$-Alkaryl bedeutet, oder

$R^3$ Alkyl mit 9 bis 25 C-Atomen, eine unsubstituierte oder $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, Alkenyl mit 5 bis 18 C-Atomen, $C_7$-$C_{18}$-Aralkyl oder $C_7$-$C_{18}$- Alkaryl bedeutet, oder

$R^1$ und $R^3$ Alkyl mit 1 bis 25 C-Atomen, eine unsubstituierte oder $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, Alkenyl mit 2 bis 18 C-Atomen, Phenyl, Naphthyl, $C_7$-$C_{18}$-Aralkyl oder $C_7$-$C_{18}$-Alkaryl, die jeweils mit einer oder mehreren Gruppen der Reihe Halogen, Cyano, Nitro, -$OR^a$ oder -$COOR^b$ substituiert sind, bedeuten, oder

$R^1$ und $R^3$ Alkyl mit 2 bis 25 C-Atomen, eine unsubstituierte oder $C_1$-$C_8$-alkylsubstituierte Cycloalkylgruppe mit 3 bis 10 Ring-C-Atomen, eine unsubstituierte oder $C_1$-$C_8$-alkylsubstituierte $C_3$-$C_{12}$-Cycloalkyl-$C_1$-$C_4$-alkylgruppe, Alkenyl mit 3 bis 18 C-Atomen, $C_7$-$C_{18}$-Aralkyl oder $C_7$-$C_{18}$-Alkaryl, die jeweils durch eine oder mehrere Gruppen der Reihe -O-, -S-, -NH-,

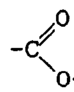 oder 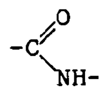

unterbrochen sind, wobei $C_7$-$C_{18}$-Aralkyl oder $C_7$-$C_{18}$-Alkaryl nur in den Alkylresten unterbrochen sind, bedeuten, oder

$R^1$ und $R^3$ Alkyl mit 2 bis 25 C-Atomen, eine unsubstituierte oder $C_1$-$C_8$-alkylsubstituierte Cycloalkylgruppe mit 3 bis 10 Ring-C-Atomen, eine unsubstituierte oder $C_1$-$C_8$-alkylsubstituierte $C_3$-$C_{12}$-Cycloalkyl-$C_1$-$C_4$-alkylgruppe, Alkenyl mit 3 bis 18 C-Atomen, $C_7$-$C_{18}$-Aralkyl oder $C_7$-$C_{18}$-Alkaryl, die jeweils durch eine oder mehrere Gruppen der Reihe Halogen, Cyano, Nitro, -$OR^a$ oder -$COOR^b$ substituiert und durch jeweils eine oder mehrere Gruppen der Reihe -O-, -S-, -NH-,

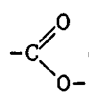 oder 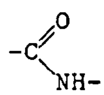

unterbrochen sind, wobei $C_7$-$C_{18}$-Aralkyl oder $C_7$-$C_{18}$-Alkaryl nur in den Alkylresten unterbrochen sind, bedeuten,

wobei $R^a$ die Bedeutung von $R^3$ mit Ausnahme von -$OR^a$ und

$R^b$ die Bedeutung von Alkyl mit 1 bis 18 C-Atomen oder von Alkyl mit 2 bis 20 C-Atomen, die durch eine oder mehrere Gruppen der Reihe -O-, -S-, -NH-,

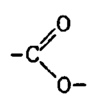 oder 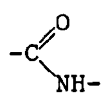

unterbrochen sind, oder einer unsubstituierten oder $C_1$-$C_8$-alkylsubstituierten Cycloalkylgruppe mit 5 bis 12 Ring-C-Atomen oder von Phenyl, von Naphthyl, von $C_7$-$C_{18}$-Alkaryl oder von $C_7$-$C_{18}$-Aralkyl hat, und

$R^6$ und $R^7$ Alkyl mit 11 bis 18 C-Atomen oder Alkyl mit 2 bis 20 C-Atomen, das durch eine oder mehrere Gruppen der Reihe -O-, -S-, -NH-,

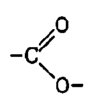 oder 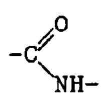

unterbrochen ist, oder eine unsubstituierte oder eine $C_1$-$C_8$-alkylsubstituierte Cycloalkylgruppe mit 5 bis 12 Ring-C-Atomen, eine unsubstituierte oder $C_1$-$C_8$-alkylsubstituierte $C_5$-$C_{12}$-Cycloalkyl-$C_1$-$C_4$-alkylgruppe, eine unsubstituierte oder $C_1$-$C_8$-alkylsubstituierte $C_3$-$C_{12}$-Cycloalkyl-$C_1$-$C_4$-alkylgruppe, die durch eine oder mehrere

Gruppen der Reihe -O-, -S-, -NH-,

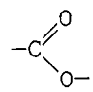 oder 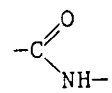

unterbrochen ist,
$C_7$-$C_{18}$-Alkaryl oder $C_7$-$C_{18}$-Aralkyl darstellen, oder,
wenn x = 2 ist,
R die Bedeutung von $R^8$ hat und
$R^8$ Alkylen mit 1 bis 18 C-Atomen, Alkylen mit 2 bis 18 C-Atomen, das durch wenigstens eine -O-Gruppe unterbrochen ist, Alkyliden mit 2 bis 20 C-Atomen oder Alkyliden mit 3 bis 20 C-Atomen, das durch wenigstens eine -O-Gruppe unterbrochen ist, darstellt,
dadurch gekennzeichnet, dass eine Verbindung der Formel

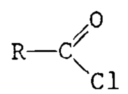 ,

mit KSCN zu einer Verbindung der Formel

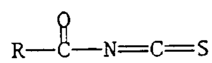

umgesetzt wird und die Verbindung

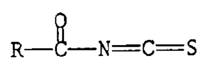

mit $HOR^6$ zu

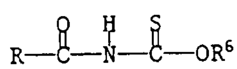

oder die Verbindung

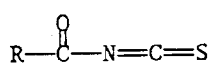

mit $HSR^7$ zu

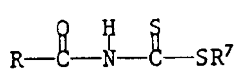

umgesetzt wird oder dass eine Verbindung der Formel

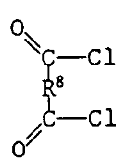 ,

mit KSCN zu

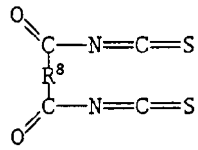

,

und diese Verbindung mit 2 Molen der Verbindung $HR^2$ zu einer Verbindung der Formel

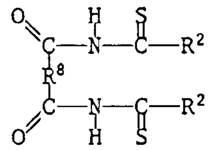

,

umgesetzt wird, wobei R, $R^2$, $R^6$, $R^7$ und $R^8$ die genannte Bedeutung haben.

**16.** Verfahren zur Herstellung von Verbindungen der Formel IIa

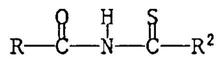 (IIa)

gemäss Anspruch 15.

**17.** Verfahren zur Herstellung von Verbindungen der Formel IIc

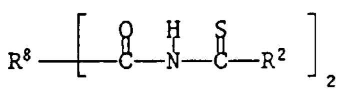 (IIc)

gemäss Anspruch 15.

**18.** Verfahren zur Herstellung von Verbindungen der Formel IIa, worin R die Bedeutung von $R^1$ oder $-O-R^3$ hat und $R^1$ Phenyl oder Alkyl mit 1 bis 18 C-Atomen und $R^3$ Alkyl mit 10 bis 18 C-Atomen oder Phenyl bedeutet und $R^2$ die Bedeutung von $-SR^7$ hat, gemäss Anspruch 16.

**19.** Verfahren zur Herstellung von Verbindungen der Formel IIa, worin $R^7$ die Bedeutung von Alkyl mit 12 bis 18 C-Atomen, $-CH_2-COO-C_1-C_{18}$-Alkyl oder Phenyl-$C_1$-$C_4$-Alkyl hat, gemäss Anspruch 18.

**20.** Verwendung der Verbindungen der Formel I gemäss Anspruch 1 und der Formel II gemäss Anspruch 15 als Hochdruck- und Verschleissschutzadditive und als Antioxidantien in Schmierstoffen und Hydraulikflüssigkeiten.

## Claims

### Claims for the following Contracting States : BE, DE, FR, GB, IT, NL

**1.** A composition comprising
a) a lubricant or a hydraulic fluid and
b) at least one compound of the general formula I

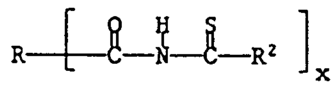

(I)

in which x = 1 or 2 and,
if x = 1,
R is as defined for $R^1$ or $R^3$-O- or,
if x = 2,
R is as defined for $R^8$, and

$R^1$ and $R^3$ are alkyl having 1 to 25 C atoms, an unsubstituted or $C_1$-$C_8$alkyl-substituted cycloalkyl group having 5 to 12 ring C atoms, an unsubstituted or $C_1$-$C_8$alkyl-substituted $C_5$-$C_{12}$cycloalkyl-$C_1$-$C_4$alkyl group, alkenyl having 2 to 18 C atoms, phenyl, naphthyl, $C_7$-$C_{18}$aralkyl or $C_7$-$C_{18}$alkaryl, or

$R^1$ and $R^3$ are alkyl having 1 to 25 C atoms, an unsubstituted or $C_1$-$C_8$alkyl-substituted cycloalkyl group having 5 to 12 ring C atoms, an unsubstituted or $C_1$-$C_8$alkyl-substituted $C_5$-$C_{12}$cycloalkyl-$C_1$-$C_4$alkyl group, alkenyl having 2 to 18 C atoms, phenyl, naphthyl, $C_7$-$C_{18}$aralkyl or $C_7$-$C_{18}$alkaryl, each of which are monosubstituted or polysubstituted by groups from the series comprising halogen, cyano, nitro, -$OR^a$ or -$COOR^b$, or $R^1$ and $R^3$ are alkyl having 2 to 25 C atoms, an unsubstituted or $C_1$-$C_8$alkyl-substituted cycloalkyl group having 3 to 10 ring C atoms, an unsubstituted or $C_1$-$C_8$alkyl-substituted $C_3$-$C_{12}$cycloalkyl-$C_1$-$C_4$alkyl group, alkenyl having 3 to 18 C atoms, $C_7$-$C_{18}$aralkyl or $C_7$-$C_{18}$alkaryl, each of which are interrupted by one or more groups from the series comprising -O-, -S-, -NH-,

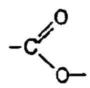 or 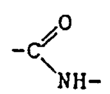 ,

$C_7$-$C_{18}$aralkyl or $C_7$-$C_{18}$alkaryl being interrupted only in the alkyl radicals, or

$R^1$ and $R^3$ are alkyl having 2 to 25 C atoms, an unsubstituted or $C_1$-$C_8$alkyl-substituted cycloalkyl group having 3 to 10 ring C atoms, an unsubstituted or $C_1$-$C_8$alkyl-substituted $C_3$-$C_{12}$cycloalkyl-$C_1$-$C_4$alkyl group, alkenyl having 3 to 18 C atoms, $C_7$-$C_{18}$aralkyl or $C_7$-$C_{18}$alkaryl, each of which are monosubstituted or polysubstituted by groups from the series comprising halogen, cyano, nitro, -$OR^a$ or -$COOR^b$ and are each interrupted by one or more groups from the series comprising -O-, -S-, -NH-,

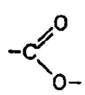 or 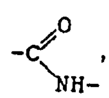 ,

$C_7$-$C_{18}$aralkyl or $C_7$-$C_{18}$alkaryl being interrupted only in the alkyl radicals, $R^a$ being as defined for $R^3$ with the exception of -$OR^a$ and
$R^b$ being as defined for $R^6$, and
$R^2$ is -$NR^4$ $R^5$, -$OR^6$ or -$SR^7$, and

$R^4$ and $R^5$ are identical or different and are -H, alkyl having 1 to 23 C atoms, an unsubstituted or $C_1$-$C_8$alkyl-substituted cycloalkyl group having 5 to 12 ring C atoms, an unsubstituted or $C_1$-$C_8$alkyl-substituted $C_5$-$C_{12}$cycloalkyl-$C_1$-$C_4$alkyl group, phenyl, naphthyl, $C_7$-$C_{18}$aralkyl or $C_7$-$C_{18}$alkaryl, or

$R^4$ and $R^5$, together with the N atom linking them, form a piperidine, morpholine, hexamethyleneimine (perhydroazepine), pyrrolidine, piperazine or 1-methylpiperazine radical, and

$R^6$ and $R^7$ are alkyl having 1 to 18 C atoms or alkyl having 2 to 20 C atoms which are interrupted by one or more groups from the series comprising -O-, -S-, -NH-,

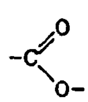 or 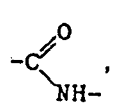 ,

or an unsubstituted or $C_1$-$C_8$alkyl-substituted cycloalkyl group having 5 to 12 ring C atoms, an unsubsti-

tuted or $C_1$-$C_8$alkyl-substituted $C_5$-$C_{12}$cycloalkyl-$C_1$-$C_4$alkyl group, an unsubstituted or $C_1$-$C_8$alkyl-substituted $C_3$-$C_{12}$cycloalkyl-$C_1$-$C_4$alkyl group which is interrupted by one or more groups from the series comprising -O-, -S-, -NH-,

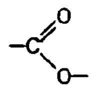 or 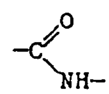,

or phenyl, naphthyl, $C_7$-$C_{18}$alkaryl or $C_7$-$C_{18}$aralkyl, and $R^8$ is alkylene having 1 to 18 C atoms, alkylene having 2 to 18 C atoms and interrupted by at least one -O- group, alkylidene having 2 to 20 C atoms or alkylidene having 3 to 20 C atoms and interrupted by at least one -O- group.

2. A composition according to claim 1, containing at least one compound of the general formula 1, in which $R^1$ and $R^3$ are alkyl having 1 to 18 C atoms, cycloalkyl having 5 to 8 ring C atoms, phenyl or $C_7$-$C_{18}$aralkyl, $R^4$ and $R^5$ are identical or different and are -H, alkyl having 1 to 18 C atoms, cycloalkyl having 5 to 12 ring C atoms or phenyl, and $R^6$ and $R^7$ are alkyl having 1 to 18 C atoms, a cycloalkyl group having 5 to 8 ring C atoms or alkyl having 2 to 20 C atoms and interrupted by one group

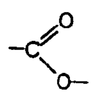

or phenyl or $C_7$-$C_{18}$aralkyl, and $R^8$ is alkylene having 1 to 18 C atoms or alkylidene having 2 to 20 C atoms.

3. A composition according to claim 1, containing a compound of the formula Ia

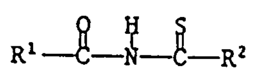

(Ia).

4. A composition according to claim 1, containing a compound of the formula Ib

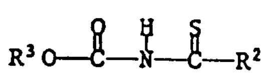

(Ib).

5. A composition according to claim 1, containing a compound of the formula Ic

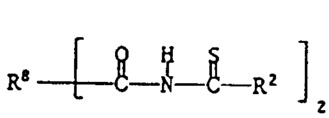

(Ic).

6. A composition according to claim 3, containing a compound of the formula Ia, in which $R^1$ is alkyl having 1 to 18 C atoms, phenyl or benzyl and $R^2$ is -O-($C_1$-$C_{18}$)alkyl, -N[($C_1$-$C_{18}$)alkyl]$_2$, -O-($C_5$-$C_{12}$)cycloalkyl, -O-phenyl, -O-($C_7$-$C_{18}$)aralkyl, -S-($C_1$-$C_{12}$)alkyl, -S-phenyl, -S-($C_7$-$C_{18}$)aralkyl or -S-$CH_2$-$COOR^9$, $R^9$ being $C_1$-$C_{18}$alkyl.

7. A composition according to claim 3, containing a compound of the formula Ia, in which $R^1$ is alkyl having 9 to 18 C atoms or phenyl and $R^2$ is -O-($C_1$-$C_{18}$)alkyl, -S-($C_8$-$C_{10}$)alkyl, -N[($C_4$-$C_8$)alkyl]$_2$, -S-$CH_2$-COO($C_2$-$C_8$)alkyl or -S-benzyl.

8. A composition according to claim 4, containing a compound of the formula Ib, in which $R^2$ is -O-($C_1$-$C_{18}$)alkyl, -O-($C_5$-$C_{12}$)cycloalkyl, -O-phenyl, -O-($C_7$-$C_{18}$)aralkyl, -S-($C_1$-$C_{12}$)alkyl, -S-phenyl, -S-($C_7$-$C_{18}$)aralkyl or -S-$CH_2$-$COOR^9$,

$R^9$ being $C_1$-$C_{18}$alkyl and
$R^3$ being $C_1$-$C_{18}$alkyl, cyclohexyl, phenyl or benzyl.

**9.** A composition according to claim 4, containing a compound of the formula Ib, in which $R^2$ is -O-($C_2$-$C_8$)alkyl, -S-($C_8$-$C_{12}$)alkyl or -N[($C_4$-$C_8$)alkyl]$_2$ and $R^3$ is $C_2$-$C_4$alkyl, cyclohexyl or phenyl.

**10.** A composition according to claim 5, containing a compound of the formula Ic, in which $R^2$ is -O-($C_1$-$C_{18}$)alkyl, -N[($C_4$-$C_8$)alkyl]$_2$, -O-phenyl, -O-($C_7$-$C_{18}$)aralkyl, -O-($C_5$-$C_{12}$)cycloalkyl, -S-($C_1$-$C_{12}$)alkyl, -S-phenyl, -S-($C_7$-$C_{18}$)aralkyl or -S-$CH_2$-$COOR^9$,
$R^9$ being $C_1$-$C_{18}$alkyl and
$R^8$ being $C_1$-$C_{10}$alkylene or $C_2$-$C_{10}$alkylidene.

**11.** A composition according to claim 5, containing a compound of the formula Ic, in which
$R^2$ is -N[($C_4$-$C_8$)alkyl]$_2$ and
$R^8$ is $C_3$-$C_8$alkylene.

**12.** A composition according to claim 3, containing a compound of the formula Ia, in which $R^1$ is phenyl and $R^2$ is $SR^7$ or -S-$CH_2COOR^9$, and $R^7$ is $C_4$-$C_{12}$alkyl and $R^9$ is $C_4$-$C_{12}$alkyl.

**13.** A composition according to claim 4, containing a compound of the formula Ib, in which $R^2$ is -$SR^7$ or -S-$CH_2COOR^9$, $R^7$ being $C_4$-$C_{12}$alkyl, $R^9$ being $C_4$-$C_{12}$alkyl and $R^3$ being $C_1$-$C_4$alkyl, cyclohexyl or phenyl.

**14.** A composition according to claim 4, containing a compound of the formula Ib, in which $R^2$ -S-n-octyl and $R^3$ is ethyl, and/or a compound of the formula Ib, in which $R^2$ is -S-(2-ethylhexyl) and $R^3$ is n-butyl.

**15.** A compound of the general formula II

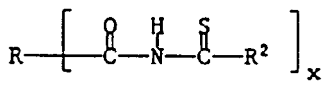

(II)

in which x = 1 or 2,
$R^2$ is as defined for -$OR^6$ or -$SR^7$ and,
if x = 1,
R is as defined for $R^1$ or $R^3$-O-,
and $R^1$ is alkyl having 1 to 25 C atoms, an unsubstituted or $C_1$-$C_8$alkyl-substituted cycloalkyl group having 5 to 12 ring C atoms, an unsubstituted or $C_1$-$C_8$alkyl-substituted $C_5$-$C_{12}$cycloalkyl-$C_1$-$C_4$alkyl group, alkenyl having 2 to 18 C atoms, phenyl, naphthyl, $C_7$-$C_{18}$aralkyl or $C_7$-$C_{18}$alkaryl, or
$R^3$ is alkyl having 9 to 25 C atoms, an unsubstituted or $C_1$-$C_8$alkyl-substituted cycloalkyl group having 5 to 12 ring C atoms, an unsubstituted or $C_1$-$C_8$alkyl-substituted $C_5$-$C_{12}$cycloalkyl-$C_1$-$C_4$alkyl group, alkenyl having 5 to 18 C atoms, $C_7$-$C_{18}$aralkyl or $C_7$-$C_{18}$alkaryl, or $R^1$ and $R^3$ are alkyl having 1 to 25 C atoms, an unsubstituted or $C_1$-$C_8$alkyl-substituted cycloalkyl group having 5 to 12 ring C atoms, an unsubstituted or $C_1$-$C_8$alkyl-substituted $C_5$-$C_{12}$cycloalkyl-$C_1$-$C_4$alkyl group, alkenyl having 2 to 18 C atoms, phenyl, naphthyl, $C_7$-$C_{18}$aralkyl or $C_7$-$C_{18}$alkaryl, each of which are monosubstituted or polysubstituted by groups from the series comprising halogen, cyano, nitro, -$OR^a$ or -$COOR^b$, or
$R^1$ and $R^3$ are alkyl having 2 to 25 C atoms, an unsubstituted or
$C_1$-$C_8$alkyl-substituted cycloalkyl group having 3 to 10 ring C atoms, an unsubstituted or $C_1$-$C_8$alkyl-substituted $C_3$-$C_{12}$cycloalkyl-$C_1$-$C_4$alkyl group, alkenyl having 3 to 18 C atoms, $C_7$-$C_{18}$aralkyl or $C_7$-$C_{18}$alkaryl, each of which are interrupted by one or more groups from the series comprising -O-, -S-, -NH-,

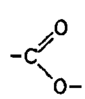 or 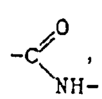 ,

$C_7$-$C_{18}$aralkyl or $C_7$-$C_{18}$alkaryl being interrupted only in the alkyl radicals, or
$R^1$ and $R^3$ are alkyl having 2 to 25 C atoms, an unsubstituted or $C_1$-$C_8$alkyl-substituted cycloalkyl group having 3 to 10 ring C atoms, an unsubstituted or $C_1$-$C_8$alkyl-substituted $C_3$-$C_{12}$cycloalkyl-$C_1$-$C_4$alkyl group, alkenyl having 3 to 18 C atoms, $C_7$-$C_{18}$aralkyl or $C_7$-$C_{18}$alkaryl, each of which are monosubstituted or polysubstituted by groups from the series comprising halogen, cyano, nitro, $OR^2$ or -$COOR^6$ and each of which

are interrupted by one or more groups from the series comprising -O-, -S-, -NH-,

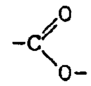 or 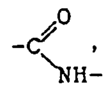 ,

$C_7$-$C_{18}$aralkyl or $C_7$-$C_{18}$alkaryl being interrupted only in the alkyl radicals,
$R^a$ being as defined for $R^3$ with the exception of -$OR^a$ and
$R^b$ being alkyl having 1 to 18 C atoms or alkyl having 2 to 20 C atoms and interrupted by one or more groups from the series comprising -O-, -S-, -NH-,

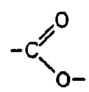 or 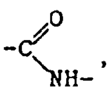 ,

or an unsubstituted or $C_1$-$C_8$alkyl-substituted cycloalkyl group having 5 to 12 ring C atoms or phenyl, naphthyl, $C_7$-$C_{18}$alkaryl or $C_7$-$C_{18}$aralkyl, and
$R^6$ and $R^7$ are alkyl having 11 to 18 C atoms or alkyl having 2 to 20 C atoms and interrupted by one or more groups from the series comprising -O-, -S-, -NH-,

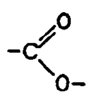 or 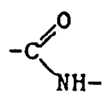 ,

or an unsubstituted or $C_1$-$C_8$alkyl-substituted cycloalkyl group having 5 to 12 ring C atoms, an unsubstituted or $C_1$-$C_8$alkyl-substituted $C_5$-$C_{12}$cycloalkyl-$C_1$-$C_4$alkyl group, an unsubstituted or $C_1$-$C_8$alkyl-substituted $C_3$-$C_{12}$cycloalkyl-$C_1$-$C_4$alkyl group interrupted by one or more groups from the series comprising -O-, -S-, -NH-,

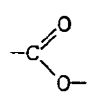 or 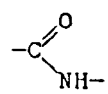 ,

$C_7$-$C_{18}$alkaryl or $C_7$-$C_{18}$aralkyl, or,
if x = 2,
R is as defined for $R^8$ and
$R^8$ is alkylene having 1 to 18 C atoms, alkylene having 2 to 18 C atoms and interrupted by at least one -O- group, alkylidene having 2 to 20 C atoms or alkylidene having 3 to 20 C atoms and interrupted by at least one -O- group.

**16.** A compound according to claim 15, of the formula IIa

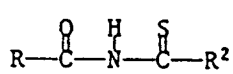 (IIa)

**17.** A compound according to claim 15, of the formula IIc

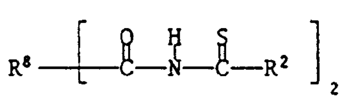 (IIc).

**18.** A compound according to claim 16, in which R is as defined for $R^1$ or -O-$R^3$ and $R^1$ is phenyl or alkyl having 1 to 18 C atoms and $R^3$ is alkyl having 10 to 18 C atoms or phenyl and $R^2$ is -$SR^7$.

**19.** A compound according to claim 18, in which $R^7$ is alkyl having 12 to 18 C atoms, -$CH_2$-COO-$C_1$-$C_{18}$alkyl or phenyl-$C_1$-$C_4$alkyl.

**20.** The use of a compound of the formula I according to claim 1 and of the formula II according to claim 15 as an extreme-pressure additive and anti-wear additive and as an antioxidant in lubricants and hydraulic fluids.

**Claims for the following Contracting State :ES**

**1.** A composition, comprising
a) a lubricant or a hydraulic fluid and
b) at least one compound of the general formula I

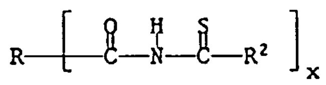

(I)

in which x = 1 or 2 and,
if x = 1,
R is as defined for $R^1$ or $R^3$-O- or,
if x = 2,
R is as defined for $R^8$, and
$R^1$ and $R^3$ are alkyl having 1 to 25 C atoms, an unsubstituted or $C_1$-$C_8$alkyl-substituted cycloalkyl group having 5 to 12 ring C atoms, an unsubstituted or $C_1$-$C_8$alkyl-substituted $C_5$-$C_{12}$cycloalkyl-$C_1$-$C_4$alkyl group, alkenyl having 2 to 18 C atoms, phenyl, naphthyl, $C_7$-$C_{18}$aralkyl or $C_7$-$C_{18}$alkaryl, or
$R^1$ and $R^3$ are alkyl having 1 to 25 C atoms, an unsubstituted or
$C_1$-$C_8$alkyl-substituted cycloalkyl group having 5 to 12 ring C atoms, an unsubstituted or $C_1$-$C_8$alkyl-substituted $C_5$-$C_{12}$cycloalkyl-$C_1$-$C_4$alkyl group, alkenyl having 2 to 18 C atoms, phenyl, naphthyl, $C_7$-$C_{18}$aralkyl or $C_7$-$C_{18}$alkaryl, each of which are monosubstituted or polysubstituted by groups from the series comprising halogen, cyano, nitro, -$OR^a$ or -$COOR^b$, or $R^1$ and $R^3$ are alkyl having 2 to 25 C atoms, an unsubstituted or
$C_1$-$C_8$alkyl-substituted cycloalkyl group having 3 to 10 ring C atoms, an unsubstituted or $C_1$-$C_8$alkyl-substituted $C_3$-$C_{12}$cycloalkyl-$C_1$-$C_4$alkyl group, alkenyl having 3 to 18 C atoms, $C_7$-$C_{18}$aralkyl or $C_7$-$C_{18}$alkaryl, each of which are interrupted by one or more groups from the series comprising -O-, -S-, -NH-,

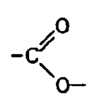 or 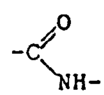 ,

$C_7$-$C_{18}$aralkyl or $C_{7-18}$alkaryl being interrupted only in the alkyl radicals, or
$R^1$ and $R^3$ are alkyl having 2 to 25 C atoms, an unsubstituted or $C_1$-$C_8$alkyl-substituted cycloalkyl group having 3 to 10 ring C atoms, an unsubstituted or $C_1$-$C_8$alkyl-substituted $C_3$-$C_{12}$cycloalkyl-$C_1$-$C_4$alkyl group, alkenyl having 3 to 18 C atoms, $C_7$-$C_{18}$aralkyl or $C_7$-$C_{18}$alkaryl, each of which are monosubstituted or polysubstituted by groups from the series comprising halogen, cyano, nitro, -$OR^a$ or -$COOR^b$ and are each interrupted by one or more groups from the series comprising -O-, -S-, -NH-,

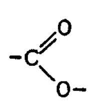 or 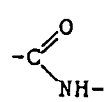 ,

$C_7$-$C_{18}$aralkyl or $C_7$-$C_{18}$alkaryl being interrupted only in the alkyl radicals, $R^a$ being as defined for $R^3$ with

the exception of $-OR^a$ and
$R^b$ being as defined for $R^6$, and
$R^2$ is $-NR^4R^5$, $-OR^6$ or $-SR^7$, and
$R^4$ and $R^5$ are identical or different and are -H, alkyl having 1 to 23 C atoms, an unsubstituted or $C_1$-$C_8$alkyl-substituted cycloalkyl group having 5 to 12 ring C atoms, an unsubstituted or $C_1$-$C_8$alkyl-substituted $C_5$-$C_{12}$cycloalkyl-$C_1$-$C_4$alkyl group, phenyl, naphthyl, $C_7$-$C_{18}$aralkyl or $C_7$-$C_{18}$alkaryl, or
$R^4$ and $R^5$, together with the N atom linking them, form a piperidine, morpholine, hexamethyleneimine (perhydroazepine), pyrrolidine, piperazine or 1-methylpiperazine radical, and $R^6$ and $R^7$ are alkyl having 1 to 18 C atoms or alkyl having 2 to 20 C atoms which are interrupted by one or more groups from the series comprising -O-, -S-, -NH-,

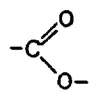 or 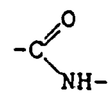 ,

or an unsubstituted or $C_1$-$C_8$alkyl-substituted cycloalkyl group having 5 to 12 ring C atoms, an unsubstituted or $C_1$-$C_8$alkyl-substituted $C_5$-$C_{12}$cycloalkyl-$C_1$-$C_4$alkyl group, an unsubstituted or $C_1$-$C_8$alkyl-substituted $C_3$-$C_{12}$cycloalkyl-$C_1$-$C_4$alkyl group which is interrupted by one or more groups from the series comprising -O-, -S-, -NH-,

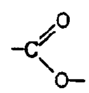 or 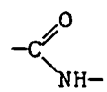 ,

or phenyl, naphthyl, $C_7$-$C_{18}$alkaryl or $C_7$-$C_{18}$aralkyl, and $R^8$ is alkylene having 1 to 18 C atoms, alkylene having 2 to 18 C atoms and interrupted by at least one -O- group, alkylidene having 2 to 20 C atoms or alkylidene having 3 to 20 C atoms and interrupted by at least one -O- group.

2. A composition according to claim 1, containing at least one compound of the general formula I, in which $R^1$ and $R^3$ are alkyl having 1 to 18 C atoms, cycloalkyl having 5 to 8 ring C atoms, phenyl or $C_7$-$C_{18}$aralkyl, $R^4$ and $R^5$ are identical or different and are -H, alkyl having 1 to 18 C atoms, cycloalkyl having 5 to 12 ring C atoms or phenyl, and $R^6$ and $R^7$ are alkyl having 1 to 18 C atoms, a cycloalkyl group having 5 to 8 ring C atoms or alkyl having 2 to 20 C atoms and interrupted by one group

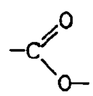

or phenyl or $C_7$-$C_{18}$aralkyl, and $R^8$ is alkylene having 1 to 18 C atoms or alkylidene having 2 to 20 C atoms.

3. A composition according to claim 1, containing a compound of the formula Ia

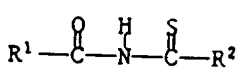 (Ia).

4. A composition according to claim 1, containing a compound of the formula Ib

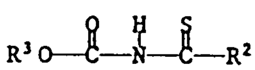 (Ib).

5. A composition according to claim 1, containing a compound of the formula Ic

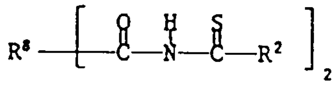 (Ic).

6. A composition according to claim 3, containing a compound of the formula Ia, in which $R^1$ is alkyl having 1 to 18 C atoms, phenyl or benzyl and $R^2$ is -O-($C_1$-$C_{18}$)alkyl, -N[($C_1$-$C_{18}$)alkyl]$_2$, -O-($C_5$-$C_{12}$)cycloalkyl, -O-phenyl, -O-($C_7$-$C_{18}$)aralkyl, -S-($C_1$-$C_{12}$)alkyl, -S-phenyl, -S-($C_7$-$C_{18}$) aralkyl or -S-$CH_2$-COOR$^9$, $R^9$ being $C_1$-$C_{18}$alkyl.

7. A composition according to claim 3, containing a compound of the formula Ia, in which $R^1$ is alkyl having 9 to 18 C atoms or phenyl and $R^2$ is -O-($C_1$-$C_{18}$)alkyl, -S-($C_8$-$C_{10}$)alkyl, -N[($C_4$-$C_8$)alkyl]$_2$, -S-$CH_2$-COO($C_2$-$C_8$)alkyl or -S-benzyl.

8. A composition according to claim 4, containing a compound of the formula Ib, in which $R^2$ is -O-($C_1$-$C_{18}$)alkyl, -O-($C_5$-$C_{12}$)cycloalkyl, -O-phenyl, -O-($C_7$-$C_{18}$)aralkyl, -S-($C_1$-$C_{12}$)alkyl, -S-phenyl, -S-($C_7$-$C_{18}$)aralkyl or -S-$CH_2$-COOR$^9$,
$R^9$ being $C_1$-$C_{18}$alkyl and
$R^3$ being $C_1$-$C_{18}$alkyl, cyclohexyl, phenyl or benzyl.

9. A composition according to claim 4, containing a compound of the formula Ib, in which $R^2$ is -O-($C_2$-$C_8$)alkyl, -S-($C_8$-$C_{12}$)alkyl or -N[($C_4$-$C_8$)alkyl]$_2$ and $R^3$ is $C_2$-$C_4$alkyl, cyclohexyl or phenyl.

10. A composition according to claim 5, containing a compound of the formula Ic, in which $R^2$ is -O-($C_1$-$C_{18}$)alkyl, -N[($C_4$-$C_8$)alkyl]$_2$, -O-phenyl, -O-($C_7$-$C_{18}$) aralkyl, -O-($C_5$-$C_{12}$)cycloalkyl, -S-($C_1$-$C_{12}$) alkyl, -S-phenyl, -S-($C_7$-$C_{18}$) aralkyl or -S-$CH_2$-COOR$^9$,
$R^9$ being $C_1$-$C_{18}$alkyl and
$R^8$ being $C_1$-$C_{10}$alkylene or $C_2$-$C_{10}$alkylidene.

11. A composition according to claim 5, containing a compound of the formula Ic, in which
$R^2$ is -N[($C_4$-$C_8$)alkyl]$_2$ and
$R^8$ is $C_3$-$C_8$alkylene.

12. A composition according to claim 3, containing a compound of the formula Ia, in which $R^1$ is phenyl and $R^2$ is SR$^7$ or -S-$CH_2$COOR$^9$, and $R^7$ is $C_4$-$C_{12}$alkyl and $R^9$ is $C_4$-$C_{12}$alkyl.

13. A composition according to claim 4, containing a compound of the formula Ib, in which $R^2$ is -SR$^7$ or -S-$CH_2$COOR$^9$, $R^7$ being $C_4$-$C_{12}$alkyl, $R^9$ being $C_4$-$C_{12}$alkyl and $R^3$ being $C_1$-$C_4$alkyl, cyclohexyl or phenyl.

14. A composition according to claim 4, containing a compound of the formula Ib, in which $R^2$ -S-n-octyl and $R^3$ is ethyl, and/or a compound of the formula Ib, in which $R^2$ is -S-(2-ethylhexyl) and $R^3$ is n-butyl.

15. A process for the preparation of a compound of the general formula II

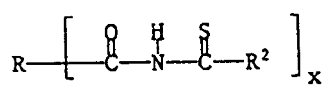

(II)

in which x = 1 or 2,
$R^2$ is as defined for -OR$^6$ or -SR$^7$ and,
if x = 1,
R is as defined for $R^1$ or $R^3$-O-,
and $R^1$ is alkyl having 1 to 25 C atoms, an unsubstituted or $C_1$-$C_8$alkyl-substituted cycloalkyl group having 5 to 12 ring C atoms, an unsubstituted or $C_1$-$C_8$alkyl-substituted $C_5$-$C_{12}$cycloalkyl-$C_1$-$C_4$alkyl group, alkenyl having 2 to 18 C atoms, phenyl, naphthyl, $C_7$-$C_{18}$aralkyl or $C_7$-$C_{18}$alkaryl, or
$R^3$ is alkyl having 9 to 25 C atoms, an unsubstituted or $C_1$-$C_8$alkyl-substituted cycloalkyl group having 5 to 12 ring C atoms, an unsubstituted or $C_1$-$C_8$alkyl-substituted $C_5$-$C_{12}$cycloalkyl-$C_1$-$C_4$alkyl group, alkenyl having 5 to 18 C atoms, $C_7$-$C_{18}$aralkyl or $C_7$-$C_{18}$alkaryl, or
$R^1$ and $R^3$ are alkyl having 1 to 25 C atoms, an unsubstituted or $C_1$-$C_8$alkyl-substituted cycloalkyl group having 5 to 12 ring C atoms, an unsubstituted or $C_1$-$C_8$alkyl-substituted $C_5$-$C_{12}$cycloalkyl-$C_1$-$C_4$alkyl group, alkenyl having 2 to 18 C atoms, phenyl, naphthyl, $C_7$-$C_{18}$aralkyl or $C_7$-$C_{18}$alkaryl, each of which are monosubstituted or polysubstituted by groups from the series comprising halogen, cyano, nitro, -OR$^a$ or -COOR$^b$, or

$R^1$ and $R^3$ are alkyl having 1 to 25 C atoms, an unsubstituted or $C_1$-$C_8$alkyl-substituted cycloalkyl group having 5 to 12 ring C atoms, an unsubstituted or $C_1$-$C_8$alkyl-substituted $C_5$-$C_{12}$cycloalkyl-$C_1$-$C_4$alkyl group, alkenyl having 2 to 18 C atoms, phenyl, naphthyl, $C_7$-$C_{18}$aralkyl or $C_7$-$C_{18}$alkaryl, each of which are monosubstituted or polysubstituted by groups from the series comprising halogen, cyano, nitro, -$OR^a$ or -$COOR^b$, or
$R^1$ and $R^3$ are alkyl having 2 to 25 C atoms, an unsubstituted or $C_1$-$C_8$alkyl-substituted cycloalkyl group having 3 to 10 ring C atoms, an unsubstituted or $C_1$-$C_8$alkyl-substituted $C_3$-$C_{12}$cycloalkyl-$C_1$-$C_4$alkyl group, alkenyl having 3 to 18 C atoms, $C_7$-$C_{18}$aralkyl or $C_7$-$C_{18}$alkaryl, each of which are interrupted by one or more groups from the series comprising -O-, -S-, -NH-,

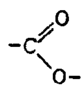 or 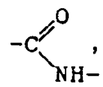 ,

$C_7$-$C_{18}$aralkyl or $C_7$-$C_{18}$alkaryl being interrupted only in the alkyl radicals, or
$R^1$ and $R^3$ are alkyl having 2 to 25 C atoms, an unsubstituted or
$C_1$-$C_8$alkyl-substituted cycloalkyl group having 3 to 10 ring C atoms, an unsubstituted or $C_1$-$C_8$alkyl-substituted $C_3$-$C_{12}$cycloalkyl-$C_1$-$C_4$alkyl group, alkenyl having 3 to 18 C atoms, $C_7$-$C_{18}$aralkyl or $C_7$-$C_{18}$alkaryl, each of which are monosubstituted or polysubstituted by groups from the series comprising halogen, cyano, nitro, $OR^2$ or -$COOR^6$ and each of which are interrupted by one or more groups from the series comprising -O-, -S-, -NH-, -O-, -S-, -NH-,

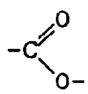 or 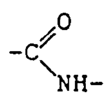 (

$C_7$-$C_{18}$aralkyl or $C_7$-$C_{18}$alkaryl being interrupted only in the alkyl radicals,
$R^a$ being as defined for $R^3$ with the exception of -$OR^a$ and
$R^b$ being alkyl having 1 to 18 C atoms or alkyl having 2 to 20 C atoms and interrupted by one or more groups from the series comprising -O-, -S-, -NH-,

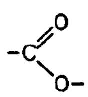 or 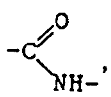 ,

or an unsubstituted or $C_1$-$C_8$alkyl-substituted cycloalkyl group having 5 to 12 ring C atoms or phenyl, naphthyl, $C_7$-$C_{18}$alkaryl or $C_7$-$C_{18}$aralkyl, and
$R^6$ and $R^7$ are alkyl having 11 to 18 C atoms or alkyl having 2 to 20 C atoms and interrupted by one or more groups from the series comprising -O-, -S-, -NH-,

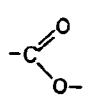 or 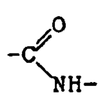 ,

or an unsubstituted or $C_1$-$C_8$alkyl-substituted cycloalkyl group having 5 to 12 ring C atoms, an unsubstituted or $C_1$-$C_8$alkyl-substituted $C_5$-$C_{12}$cycloalkyl-$C_1$-$C_4$alkyl group, an unsubstituted or $C_1$-$C_8$alkyl-substituted $C_3$-$C_{12}$cycloalkyl-$C_1$-$C_4$alkyl group interrupted by one or more groups from the series comprising -O-, -S-, -NH-,

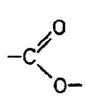 or 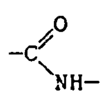 ,

$C_7$-$C_{18}$alkaryl or $C_7$-$C_{18}$aralkyl, or,

if x = 2,
R is as defined for $R^8$ and
$R^8$ is alkylene having 1 to 18 C atoms, alkylene having 2 to 18 C atoms and interrupted by at least one -O- group, alkylidene having 2 to 20 C atoms or alkylidene having 3 to 20 C atoms and interrupted by at least one -O- group,
which comprises reacting a compound of the formula

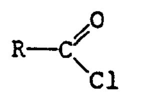,

with KSCN to give a compound of the formula

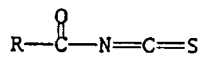

and reacting the compound

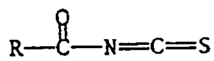

with $HOR^6$ to give

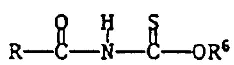

or the compound

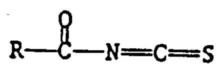

with $HSR^7$ to give

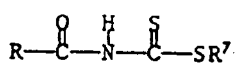

or which comprises reacting a compound of the formula

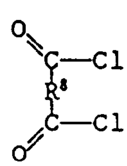

with KSCN to give

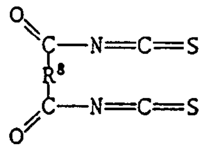

,

and reacting this compound with 2 moles of the compound $HR^2$ to give a compound of the formula

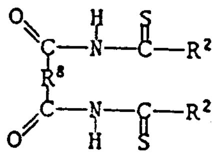

,

in which R, $R^2$, $R^6$, $R^7$ and $R^8$ are as defined above.

**16.** A process for the preparation of a compound of the formula IIa

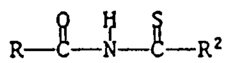

(IIa)

according to claim 15.

**17.** A process for the preparation of a compound of the formula IIc

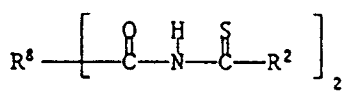

(IIc)

according to claim 15.

**18.** A process for the preparation of a compound of the formula IIa in which R is as defined for $R^1$ or $-O-R^3$ and $R^1$ is phenyl or alkyl having 1 to 18 C atoms and $R^3$ is alkyl having 10 to 18 C atoms or phenyl and $R^2$ is $-SR^7$ according to claim 16.

**19.** A process for the preparation of a compound of the formula IIa in which $R^7$ is alkyl having 12 to 18 C atoms, $-CH_2-COO-C_1-C_{18}$alkyl or phenyl-$C_1-C_4$alkyl, according to claim 18.

**20.** The use of a compound of the formula I according to claim 1 and of the formula II according to claim 15 as an extreme-pressure additive and anti-wear additive and as an antioxidant in lubricants and hydraulic fluids.

**Revendications**

**Revendications pour les Etats contractants suivants : BE, DE, FR, GB, IT, NL**

**1.** Composition contenant

a) un lubrifiant ou un fluide hydraulique et

b) au moins un composé de formule générale I

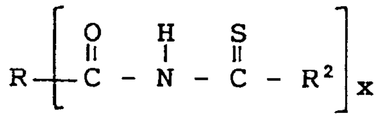 (I)

dans laquelle x = 1 ou 2 et,
quand x = 1,
R signifie $R^1$ ou $R^3$-O-, ou
quand x = 2,
R signifie $R^8$, et
$R^1$ et $R^3$ signifient alkyle de 1 à 25 atomes de carbone, un groupe cycloalkyle de 5 à 12 atomes de carbone cycliques non substitué ou substitué par alkyle en $C_1$-$C_8$, un groupe (cycloalkyl en $C_5$-$C_{12}$)-(alkyle en $C_1$-$C_4$) non substitué ou substitué par alkyle en $C_1$-$C_8$, alcényle de 2 à 18 atomes de carbone, phényle, naphtyle, aralkyle en $C_7$-$C_{18}$ ou alkaryle en $C_7$-$C_{18}$ ou bien
$R^1$ et $R^3$ signifient alkyle de 1 à 25 atomes de carbone, un groupe cycloalkyle de 5 à 12 atomes de carbone cycliques non substitué ou substitué par alkyle en $C_1$-$C_8$, un groupe (cycloalkyl en $C_5$-$C_{12}$)-(alkyle en $C_1$-$C_4$) non substitué ou substitué par alkyle en $C_1$-$C_8$, alcényle de 2 à 18 atomes de carbone, phényle, naphtyle, aralkyle en $C_7$-$C_{18}$ ou alkaryle en $C_7$-$C_{18}$, qui sont à chaque fois substitués par un ou plusieurs groupes de la série halogène, cyano, nitro, -$OR^a$ ou -$COOR^b$, ou bien
$R^1$ et $R^3$ signifient alkyle de 2 à 25 atomes de carbone, un groupe cycloalkyle de 3 à 10 atomes de carbone cycliques non substitué ou substitué par alkyle en $C_1$-$C_8$, un groupe (cycloalkyl en $C_3$-$C_{12}$)-(alkyle en $C_1$-$C_4$) non substitué ou substitué par alkyle en $C_1$-$C_8$, alcényle de 3 à 18 atomes de carbone, aralkyle en $C_7$-$C_{18}$ ou alkaryle en $C_7$-$C_{18}$, qui sont interrompus à chaque fois par un ou plusieurs groupes de la série
-O-, -S-, -NH-,

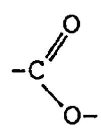 ou 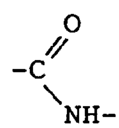 ,

les aralkyles en $C_7$-$C_{18}$ ou les alkaryles en $C_7$-$C_{18}$ n'étant interrompus que dans les restes alkyle, ou bien
$R^1$ et $R^3$ signifient alkyle de 2 à 25 atomes de carbone, un groupe cycloalkyle de 3 à 10 atomes de carbone cycliques non substitué ou substitué par alkyle en $C_1$-$C_8$, un groupe (cycloalkyl en $C_3$-$C_{12}$)-(alkyle en $C_1$-$C_4$) non substitué ou substitué par alkyle en $C_1$-$C_8$, alcényle de 3 à 18 atomes de carbone, aralkyle en $C_7$-$C_{18}$ ou alkaryle en $C_7$-$C_{18}$, qui sont à chaque fois substitués par un ou plusieurs groupes de la série halogène, cyano, nitro, -$OR^a$ ou -$COOR^b$ et sont interrompus à chaque fois par un ou plusieurs groupes de la série
-O-, -S-, -NH-,

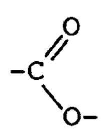 ou 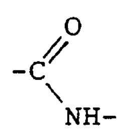 ,

les aralkyles en $C_7$-$C_{18}$ ou les alkaryles en $C_7$-$C_{18}$ n'étant interrompus que dans les restes alkyle,
où $R^a$ a la signification de $R^3$ à l'exception de -$OR^a$ et
$R^b$ a la signification de $R^6$, et
$R^2$ signifie -$NR^4R^5$, -$OR^6$ ou -$SR^7$, et
$R^4$ et $R^5$ sont identiques ou différents et signifient -H, alkyle de 1 à 23 atomes de carbone, un groupe cycloalkyle de 5 à 12 atomes de carbone cycliques non substitué ou substitué par alkyle en $C_1$-$C_8$, un groupe (cycloalkyl en $C_5$-$C_{12}$)-(alkyle en $C_1$-$C_4$) non substitué ou substitué par alkyle en $C_1$-$C_8$, phényle, naphtyle, aralkyle en $C_7$-$C_{18}$ ou alkaryle en $C_7$-$C_{18}$, ou
$R^4$ et $R^5$ forment ensemble avec l'atome d'azote auquel ils sont liés un reste de pipéridine, morpholine, hexaméthylèneimine (perhydroazépine), pyrrolidine, pipérazine ou 1-méthylpipérazine, et
$R^6$ et $R^7$ signifient alkyle de 1 à 18 atomes de carbone, ou alkyle de 2 à 20 atomes de carbone qui est

interrompu par un ou plusieurs groupes de la série -O-, -S-, -NH-,

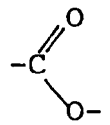 ou 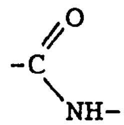 ,

ou un groupe cycloalkyle de 5 à 12 atomes de carbone cycliques non substitué ou substitué par alkyle en $C_1$-$C_8$, un groupe (cycloalkyl en $C_5$-$C_{12}$)-(alkyle en $C_1$-$C_4$) non substitué ou substitué par alkyle en $C_1$-$C_8$, ou bien un groupe (cycloalkyl en $C_3$-$C_{12}$)-(alkyle en $C_1$-$C_4$) non substitué ou substitué par alkyle en $C_1$-$C_8$, qui est interrompu par un ou plusieurs groupes de la série -O-, -S-, -NH-,

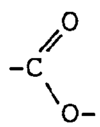 ou 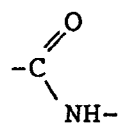 ,

ou phényle, naphtyle, alkaryle en $C_7$-$C_{18}$ ou aralkyle en $C_7$-$C_{18}$, et
$R^8$ signifie alkylène de 1 à 18 atomes de carbone, alkylène de 2 à 18 atomes de carbone qui est interrompu par au moins un groupe -O-, alkylidène de 2 à 20 atomes de carbone ou alkylidène de 3 à 20 atomes de carbone qui est interrompu par au moins un groupe -O-.

2. Compositions selon la revendication 1 contenant au moins un composé de formule générale I dans laquelle $R^1$ et $R^3$ sont alkyle de 1 à 18 atomes de carbone, cycloalkyle de 5 à 8 atomes de carbone cycliques, phényle ou aralkyle en $C_7$-$C_{18}$, $R^4$ et $R^5$ sont identiques ou différents et signifient -H, alkyle de 1 à 18 atomes de carbone, cycloalkyle de 5 à 12 atomes de carbone cycliques ou phényle, et $R^6$ et $R^7$ sont alkyle de 1 à 18 atomes de carbone, un groupe cycloalkyle de 5 à 8 atomes de carbone cycliques ou alkyle de 2 à 20 atomes de carbone qui est interrompu par un groupe

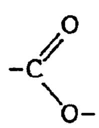

ou phényle ou aralkyle en $C_7$-$C_{18}$, et $R^8$ signifie alkylène de 1 à 18 atomes de carbone ou alkylidène de 2 à 20 atomes de carbone.

3. Composition selon la revendication 1, contenant un composé de formule Ia

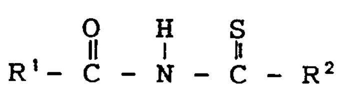 (Ia).

4. Composition selon la revendication 1, contenant un composé de formule Ib

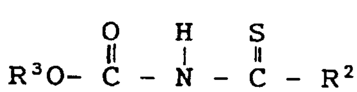 (Ib),

5. Composition selon la revendication 1, contenant un composé de formule Ic

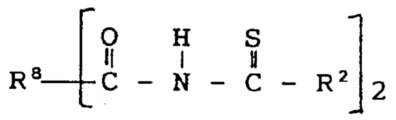 (Ic),

6. Composition selon la revendication 3, contenant un composé de formule Ia dans laquelle $R^1$ est alkyle de 1 à 18 atomes de carbone, phényle ou benzyle et $R^2$ est -O-alkyle en $C_1$-$C_{18}$, -N(alkyle en $C_1$-$C_{18}$)$_2$, -O-cycloalkyle en $C_5$-$C_{12}$, -O-phényle, -O-aralkyle en $C_7$-$C_{18}$, -S-alkyle en $C_1$-$C_{12}$, -S-phényle, -S-aralkyle en $C_7$-$C_{18}$ ou -S-$CH_2$-$COOR^9$, où $R^9$ est alkyle en $C_1$-$C_{18}$.

7. Composition selon la revendication 3, contenant un composé de formule Ia dans laquelle
$R^1$ est alkyle de 9 à 18 atomes de carbone ou phényle,
$R^2$ signifie -O-alkyle en $C_1$-$C_8$, -S-alkyle en $C_8$-$C_{10}$, -N(alkyle en $C_4$-$C_8$)$_2$, -S-$CH_2$-COO-(alkyle en $C_2$-$C_8$) ou -S-benzyle.

8. Composition selon la revendication 4, contenant un composé de formule Ib dans laquelle
$R^2$ est -O-alkyle en $C_1$-$C_{18}$, -O-cycloalkyle en $C_5$-$C_{12}$, -O-phényle, -O-aralkyle en $C_7$-$C_{18}$, -S-alkyle en $C_1$-$C_{12}$, -S-phényle, -S-aralkyle en $C_7$-$C_{18}$ ou -S-$CH_2$-$COOR^9$, où
$R^9$ est alkyle en $C_1$-$C_{18}$ et
$R^3$ est alkyle en $C_1$-$C_{18}$, cyclohexyle, phényle ou benzyle.

9. Composition selon la revendication 3, contenant un composé de formule Ib dans laquelle $R^2$ signifie -O-alkyle en $C_2$-$C_8$, -S-alkyle en $C_8$-$C_{12}$ ou -N(alkyle en $C_4$-$C_8$)$_2$ et
$R^3$ est alkyle en $C_2$-$C_4$, cyclohexyle ou phényle.

10. Composition selon la revendication 5, contenant un composé de formule Ic dans laquelle
$R^2$ est -O-alkyle en $C_1$-$C_{18}$, -N(alkyle en $C_4$-$C_8$)$_2$, -O-phényle, -O-aralkyle en $C_7$-$C_{18}$, -O-cycloalkyle en $C_5$-$C_{12}$, -S-alkyle en $C_1$-$C_{12}$, -S-phényle, -S-aralkyle en $C_7$-$C_{18}$ ou -S-$CH_2$-$COOR^9$, où
$R^9$ est alkyle en $C_1$-$C_{18}$, et
$R^8$ est alkylène en $C_1$-$C_{10}$ ou alkylidène en $C_2$-$C_{10}$.

11. Composition selon la revendication 5, contenant un composé de formule Ic dans laquelle
$R^2$ signifie -N(alkyle en $C_4$-$C_8$)$_2$ et
$R^8$ est alkylène en $C_3$-$C_8$.

12. Composition selon la revendication 3, contenant un composé de formule Ia dans laquelle $R^1$ est phényle et $R^2$ est -$SR^7$ ou -S-$CH_2$-$COOR^9$, et $R^7$ signifie alkyle en $C_4$-$C_{12}$ et $R^9$ alkyle en $C_4$-$C_{12}$.

13. Composition selon la revendication 4, contenant un composé de formule Ib dans laquelle
$R^2$ est -$SR^7$ ou -S-$CH_2$-$COOR^9$, où $R^7$ signifie alkyle en $C_4$-$C_{12}$ et $R^9$ alkyle en $C_4$-$C_{12}$, et
$R^3$ est alkyle en $C_1$-$C_4$, cyclohexyle ou phényle.

14. Composition selon la revendication 4, contenant un composé de formule Ib dans laquelle $R^2$ est -S-n-octyle et $R^3$ est éthyle, et/ou un composé de formule Ib dans laquelle $R^2$ est -S-(2-éthylhexyle) et $R^3$ est n-butyle.

15. Composés de formule générale II

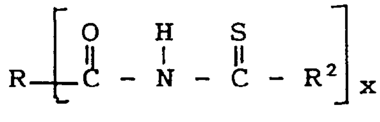 (II)

dans laquelle x = 1 ou 2,
$R^2$ signifie -$OR^6$ ou -$SR^7$, et
quand x = 1,
R signifie $R^1$ ou $R^3$-O-,

et $R^1$ signifie alkyle de 1 à 25 atomes de carbone, un groupe cycloalkyle de 5 à 12 atomes de carbone cycliques non substitué ou substitué par alkyle en $C_1$-$C_8$, un groupe (cycloalkyl en $C_5$-$C_{12}$)-(alkyle en $C_1$-$C_4$) non substitué ou substitué par alkyle en $C_1$-$C_8$, alcényle de 2 à 18 atomes de carbone, phényle, naphtyle, aralkyle en $C_7$-$C_{18}$ ou alkaryle en $C_7$-$C_{18}$ ou
$R^3$ signifie alkyle de 9 à 25 atomes de carbone, un groupe cycloalkyle de 5 à 12 atomes de carbone cycliques non substitué ou substitué par alkyle en $C_1$-$C_8$, un groupe (cycloalkyl en $C_5$-$C_{12}$)-(alkyle en $C_1$-$C_4$) non substitué ou substitué par alkyle en $C_1$-$C_8$, alcényle de 5 à 18 atomes de carbone, aralkyle en $C_7$-$C_{18}$ ou alkaryle en $C_7$-$C_{18}$ ou
$R^1$ et $R^3$ signifient alkyle de 1 à 25 atomes de carbone, un groupe cycloalkyle de 5 à 12 atomes de carbone cycliques non substitué ou substitué par alkyle en $C_1$-$C_8$, un groupe (cycloalkyl en $C_5$-$C_{12}$)-(alkyle en $C_1$-$C_4$) non substitué ou substitué par alkyle en $C_1$-$C_8$, alcényle de 2 à 18 atomes de carbone, phényle, naphtyle, aralkyle en $C_7$-$C_{18}$ ou alkaryle en $C_7$-$C_{18}$, qui sont à chaque fois substitués par un ou plusieurs groupes de la série halogène, cyano, nitro, -$OR^a$ ou -$COOR^b$, ou bien
$R^1$ et $R^3$ signifient alkyle de 2 à 25 atomes de carbone, un groupe cycloalkyle de 3 à 10 atomes de carbone cycliques non substitué ou substitué par alkyle en $C_1$-$C_8$, un groupe (cycloalkyl en $C_3$-$C_{12}$)-(alkyle en $C_1$-$C_4$) non substitué ou substitué par alkyle en $C_1$-$C_8$, alcényle de 3 à 18 atomes de carbone, aralkyle en $C_7$-$C_{18}$ ou alkaryle en $C_7$-$C_{18}$, qui sont interrompus à chaque fois par un ou plusieurs groupes de la série
-O-, -S-, -NH-,

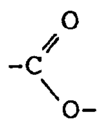 ou 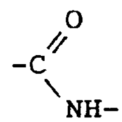 ,

les aralkyles en $C_7$-$C_{18}$ ou les alkaryles en $C_7$-$C_{18}$ n'étant interrompus que dans les restes alkyle, ou bien
$R^1$ et $R^3$ signifient alkyle de 2 à 25 atomes de carbone, un groupe cycloalkyle de 3 à 10 atomes de carbone cycliques non substitué ou substitué par alkyle en $C_1$-$C_8$, un groupe (cycloalkyl en $C_3$-$C_{12}$)-(alkyle en $C_1$-$C_4$) non substitué ou substitué par alkyle en $C_1$-$C_8$, alcényle de 3 à 18 atomes de carbone, aralkyle en $C_7$-$C_{18}$ ou alkaryle en $C_7$-$C_{18}$, qui sont à chaque fois substitués par un ou plusieurs groupes de la série halogène, cyano, nitro, -$OR^a$ ou -$COOR^b$ et sont interrompus à chaque fois par un ou plusieurs groupes de la série
-O-, -S-, -NH-,

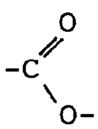 ou 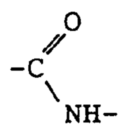 ,

les aralkyles en $C_7$-$C_{18}$ ou les alkaryles en $C_7$-$C_{18}$ n'étant interrompus que dans les restes alkyle,
où $R^a$ a la signification de $R^3$ à l'exception de -$OR^a$ et
$R^b$ signifie alkyle de 1 à 18 atomes de carbone ou alkyle de 2 à 20 atomes de carbone interrompu par un ou plusieurs groupes de la série
-O-, -S-, -NH-,

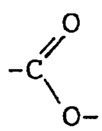 ou 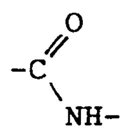 ,

ou un groupe cycloalkyle de 5 à 12 atomes de carbone cycliques non substitué ou substitué par alkyle en $C_1$-$C_8$, phényle, naphtyle, aralkyle en $C_7$-$C_{18}$ ou alkaryle en $C_7$-$C_{18}$, et $R^6$ et $R^7$ signifient alkyle de 11 à 18 atomes de carbone, ou allyle de 2 à 20 atomes de carbone qui est interrompu par un ou plusieurs groupes de la série
-O-, -S-, -NH-,

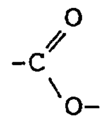 ou 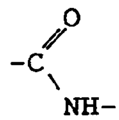 ,

ou un groupe cycloalkyle de 5 à 12 atomes de carbone cycliques non substitué ou substitué par alkyle en $C_1$-$C_8$, un groupe (cycloalkyl en $C_5$-$C_{12}$)-(alkyle en $C_1$-$C_4$) non substitué ou substitué par alkyle en $C_1$-$C_8$, ou bien un groupe (cycloalkyl en $C_3$-$C_{12}$)-(alkyle en $C_1$-$C_4$) non substitué ou substitué par alkyle en $C_1$-$C_8$, qui est interrompu par un ou plusieurs groupes de la série
-O-, -S-, -NH-,

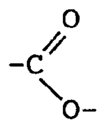 ou 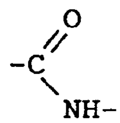 ,

ou alkaryle en $C_7$-$C_{18}$ ou aralkyle en $C_7$-$C_{18}$, ou bien,
quand x = 2,
R a la signification de $R^8$ et
$R^8$ signifie alkylène de 1 à 18 atomes de carbone, alkylène de 2 à 18 atomes de carbone qui est interrompu par au moins un groupe -O-, alkylidène de 2 à 20 atomes de carbone ou alkylidène de 3 à 20 atomes de carbone qui est interrompu par au moins un groupe -O-.

**16.** Composés selon la revendication 15 de formule IIa

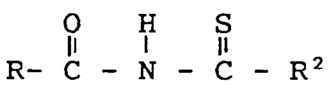 (IIa),

**17.** Composés selon la revendication 15, de formule IIc

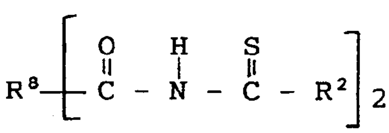 (IIc),

**18.** Composés selon la revendication 16, dans lesquels R signifie $R^1$ ou -O-$R^3$ et $R^1$ est phényle ou alkyle de 1 à 18 atomes de carbone et $R^3$ est alkyle de 10 à 18 atomes de carbone ou phényle, et $R^2$ signifie -$SR^7$.

**19.** Composés selon la revendication 18, dans lesquels $R^7$ signifie alkyle de 12 à 18 atomes de carbone, -$CH_2$-COO-(alkyle en $C_1$-$C_{18}$) ou phényl-(alkyle en $C_1$-$C_4$).

**20.** Utilisation des composés de formule I selon la revendication 1 et de formule II selon la revendication 15 comme additifs d'extrême pression et anti-usure et comme antioxydants dans des lubrifiants et des fluides hydrauliques.

**Revendications pour l'Etat contractant suivant : ES**

**1.** Composition contenant
a) un lubrifiant ou un fluide hydraulique et
b) au moins un composé de formule générale I

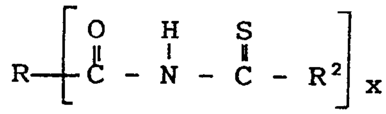 (I)

dans laquelle x = 1 ou 2 et,
quand x = 1,
R signifie $R^1$ ou $R^3$-O-, ou
quand x = 2,
R signifie $R^8$, et
$R^1$ et $R^3$ signifient alkyle de 1 à 25 atomes de carbone, un groupe cycloalkyle de 5 à 12 atomes de carbone cycliques non substitué ou substitué par alkyle en $C_1$-$C_8$, un groupe (cycloalkyl en $C_5$-$C_{12}$)-(alkyle en $C_1$-$C_4$) non substitué ou substitué par alkyle en $C_1$-$C_8$, alcényle de 2 à 18 atomes de carbone, phényle, naphtyle, aralkyle en $C_7$-$C_{18}$ ou alkaryle en $C_7$-$C_{18}$ ou bien
$R^1$ et $R^3$ signifient alkyle de 1 à 25 atomes de carbone, un groupe cycloalkyle de 5 à 12 atomes de carbone cycliques non substitué ou substitué par alkyle en $C_1$-$C_8$, un groupe (cycloalkyl en $C_5$-$C_{12}$)-(alkyle en $C_1$-$C_4$) non substitué ou substitué par allyle en $C_1$-$C_8$, alcényle de 2 à 18 atomes de carbone, phényle, naphtyle, aralkyle en $C_7$-$C_{18}$ ou alkaryle en $C_7$-$C_{18}$, qui sont à chaque fois substitués par un ou plusieurs groupes de la série halogène, cyano, nitro, -$OR^a$ ou -$COOR^b$, ou bien
$R^1$ et $R^3$ signifient alkyle de 2 à 25 atomes de carbone, un groupe cycloalkyle de 3 à 10 atomes de carbone cycliques non substitué ou substitué par alkyle en $C_1$-$C_8$, un groupe (cycloalkyl en $C_3$-$C_{12}$)-(alkyle en $C_1$-$C_4$) non substitué ou substitué par alkyle en $C_1$-$C_8$, alcényle de 3 à 18 atomes de carbone, aralkyle en $C_7$-$C_{18}$ ou alkaryle en $C_7$-$C_{18}$, qui sont interrompus à chaque fois par un ou plusieurs groupes de la série -O-, -S-, -NH-,

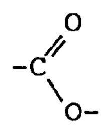 ou 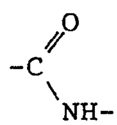 ,

les aralkyles en $C_7$-$C_{18}$ ou les alkaryles en $C_7$-$C_{18}$ n'étant interrompus que dans les restes allyle, ou bien
$R^1$ et $R^3$ signifient alkyle de 2 à 25 atomes de carbone, un groupe cycloalkyle de 3 à 10 atomes de carbone cycliques non substitué ou substitué par alkyle en $C_1$-$C_8$, un groupe (cycloalkyl en $C_3$-$C_{12}$)-(alkyle en $C_1$-$C_4$) non substitué ou substitué par alkyle en $C_1$-$C_8$, alcényle de 3 à 18 atomes de carbone, aralkyle en $C_7$-$C_{18}$ ou alkaryle en $C_7$-$C_{18}$, qui sont à chaque fois substitués par un ou plusieurs groupes de la série halogène, cyano, nitro, -$OR^a$ ou -$COOR^b$ et sont interrompus à chaque fois par un ou plusieurs groupes de la série -O-, -S-, -NH-,

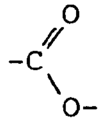 ou 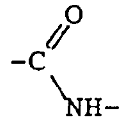 ,

les aralkyles en $C_7$-$C_{18}$ ou les alkaryles en $C_7$-$C_{18}$ n'étant interrompus que dans les restes alkyle,
où $R^a$ a la signification de $R^3$ à l'exception de -$OR^a$ et
$R^b$ a la signification de $R^6$, et
$R^2$ signifie -$NR^4R^5$, -$OR^6$ ou -$SR^7$, et
$R^4$ et $R^5$ sont identiques ou différents et signifient -H, alkyle de 1 à 23 atomes de carbone, un groupe cycloalkyle de 5 à 12 atomes de carbone cycliques non substitué ou substitué par alkyle en $C_1$-$C_8$, un groupe (cycloalkyl en $C_5$-$C_{12}$)-(alkyle en $C_1$-$C_4$) non substitué ou substitué par alkyle en $C_1$-$C_8$, phényle, naphtyle, aralkyle en $C_7$-$C_{18}$ ou alkaryle en $C_7$-$C_{18}$, ou
$R^4$ et $R^5$ forment ensemble avec l'atome d'azote auquel ils sont liés un reste de pipéridine, morpholine, hexaméthylèneimine (perhydroazépine), pyrrolidine, pipérazine ou 1-méthylpipérazine, et
$R^6$ et $R^7$ signifient alkyle de 1 à 18 atomes de carbone, ou alkyle de 2 à 20 atomes de carbone qui est interrompu par un ou plusieurs groupes de la série -O-, -S-, -NH-,

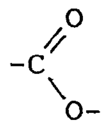 ou 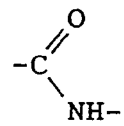 ,

ou un groupe cycloalkyle de 5 à 12 atomes de carbone cycliques non substitué ou substitué par alkyle en $C_1$-$C_8$, un groupe (cycloalkyl en $C_5$-$C_{12}$)-(alkyle en $C_1$-$C_4$) non substitué ou substitué par alkyle en $C_1$-$C_8$, ou bien un groupe (cycloalkyl en $C_3$-$C_{12}$)-(alkyle en $C_1$-$C_4$) non substitué ou substitué par alkyle en $C_1$-$C_8$, qui est interrompu par un ou plusieurs groupes de la série -O-, -S-, -NH-,

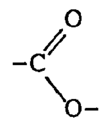 ou 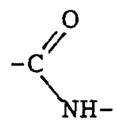 ,

ou phényle, naphtyle, alkaryle en $C_7$-$C_{18}$ ou aralkyle en $C_7$-$C_{18}$, et
$R^8$ signifie alkylène de 1 à 18 atomes de carbone, alkylène de 2 à 18 atomes de carbone qui est interrompu par au moins un groupe -O-, alkylidène de 2 à 20 atomes de carbone ou alkylidène de 3 à 20 atomes de carbone qui est interrompu par au moins un groupe -O-.

**2.** Compositions selon la revendication 1 contenant au moins un composé de formule générale I dans laquelle $R^1$ et $R^3$ sont alkyle de 1 à 18 atomes de carbone, cycloalkyle de 5 à 8 atomes de carbone cycliques, phényle ou aralkyle en $C_7$-$C_{18}$, $R^4$ et $R^5$ sont identiques ou différents et signifient -H, alkyle de 1 à 18 atomes de carbone, cycloalkyle de 5 à 12 atomes de carbone cycliques ou phényle, et $R^6$ et $R^7$ sont alkyle de 1 à 18 atomes de carbone, un groupe cycloalkyle de 5 à 8 atomes de carbone cycliques ou alkyle de 2 à 20 atomes de carbone qui est interrompu par un groupe

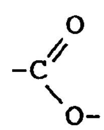

ou phényle ou aralkyle en $C_7$-$C_{18}$, et $R^8$ signifie alkylène de 1 à 18 atomes de carbone ou alkylidène de 2 à 20 atomes de carbone.

**3.** Composition selon la revendication 1, contenant un composé de formule Ia

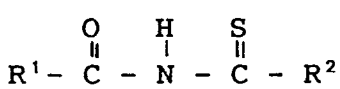 (Ia).

**4.** Composition selon la revendication 1, contenant un composé de formule Ib

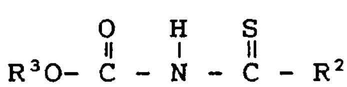 (Ib),

**5.** Composition selon la revendication 1, contenant un composé de formule Ic

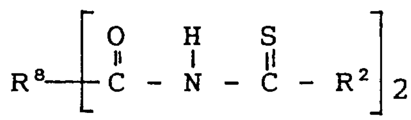

(Ic),

6. Composition selon la revendication 3, contenant un composé de formule Ia dans laquelle $R^1$ est alkyle de 1 à 18 atomes de carbone, phényle ou benzyle et $R^2$ est -O-alkyle en $C_1$-$C_{18}$, -N(alkyle en $C_1$-$C_{18}$)$_2$, -O-cycloalkyle en $C_5$-$C_{12}$, -O-phényle, -O-aralkyle en $C_7$-$C_{18}$, -S-allyle en $C_1$-$C_{12}$, -S-phényle, -S-aralkyle en $C_7$-$C_{18}$ ou -S-$CH_2$-$COOR^9$, où $R^9$ est alkyle en $C_1$-$C_{18}$.

7. Composition selon la revendication 3, contenant un composé de formule Ia dans laquelle
$R^1$ est alkyle de 9 à 18 atomes de carbone ou phényle,
$R^2$ signifie -O-alkyle en $C_1$-$C_8$, -S-alkyle en $C_8$-$C_{10}$, -N(alkyle en $C_4$-$C_8$)$_2$, -S-$CH_2$-COO-(alkyle en $C_2$-$C_8$) ou -S-benzyle.

8. Composition selon la revendication 4, contenant un composé de formule Ib dans laquelle
$R^2$ est -O-alkyle en $C_1$-$C_{18}$, -O-cycloalkyle en $C_5$-$C_{12}$, -O-phényle, -O-aralkyle en $C_7$-$C_{18}$, -S-alkyle en $C_1$-$C_{12}$, -S-phényle, -S-aralkyle en $C_7$-$C_{18}$ ou -S-$CH_2$-$COOR^9$,
où $R^9$ est alkyle en $C_1$-$C_{18}$ et
$R^3$ est alkyle en $C_1$-$C_{18}$, cyclohexyle, phényle ou benzyle.

9. Composition selon la revendication 3, contenant un composé de formule Ib dans laquelle $R^2$ signifie -O-alkyle en $C_2$-$C_8$, -S-alkyle en $C_8$-$C_{12}$ ou -N(alkyle en $C_4$-$C_8$)$_2$ et
$R^3$ est alkyle en $C_2$-$C_4$, cyclohexyle ou phényle.

10. Composition selon la revendication 5, contenant un composé de formule Ic dans laquelle
$R^2$ est -O-alkyle en $C_1$-$C_{18}$, -N(alkyle en $C_4$-$C_8$)$_2$, -O-phényle, -O-aralkyle en $C_7$-$C_{18}$, -O-cycloalkyle en $C_5$-$C_{12}$, -S-alkyle en $C_1$-$C_{12}$, -S-phényle, -S-aralkyle en $C_7$-$C_{18}$ ou -S-$CH_2$-$COOR^9$, où
$R^9$ est alkyle en $C_1$-$C_{18}$, et
$R^8$ est alkylène en $C_1$-$C_{10}$ ou alkylidène en $C_2$-$C_{10}$.

11. Composition selon la revendication 5, contenant un composé de formule Ic dans laquelle
$R^2$ signifie -N(alkyle en $C_4$-$C_8$)$_2$ et
$R^8$ est alkylène en $C_3$-$C_8$.

12. Composition selon la revendication 3, contenant un composé de formule Ia dans laquelle $R^1$ est phényle et $R^2$ est -$SR^7$ ou -S-$CH_2$-$COOR^9$, et $R^7$ signifie alkyle en $C_4$-$C_{12}$ et $R^9$ alkyle en $C_4$-$C_{12}$.

13. Composition selon la revendication 4, contenant un composé de formule Ib dans laquelle
$R^2$ est -$SR^7$ ou -S-$CH_2$-$COOR^9$, où $R^7$ signifie alkyle en $C_4$-$C_{12}$ et $R^9$ alkyle en $C_4$-$C_{12}$, et
$R^3$ est alkyle en $C_1$-$C_4$, cyclohexyle ou phényle.

14. Composition selon la revendication 4, contenant un composé de formule Ib dans laquelle $R^2$ est -S-n-octyle et $R^3$ est éthyle, et/ou un composé de formule Ib dans laquelle $R^2$ est -S-(2-éthylhexyle) et $R^3$ est n-butyle.

15. Procédé de préparation de composés de formule générale II

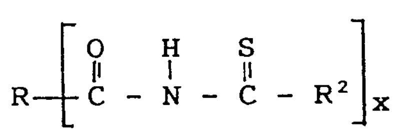

(II)

dans laquelle x = 1 ou 2,
$R^2$ signifie -$OR^6$ ou -$SR^7$, et
quand x = 1,

R signifie $R^1$ ou $R^3$-O-,

et $R^1$ signifie alkyle de 1 à 25 atomes de carbone, un groupe cycloalkyle de 5 à 12 atomes de carbone cycliques non substitué ou substitué par alkyle en $C_1$-$C_8$, un groupe (cycloalkyl en $C_5$-$C_{12}$)-(alkyle en $C_1$-$C_4$) non substitué ou substitué par alkyle en $C_1$-$C_8$, alcényle de 2 à 18 atomes de carbone, phényle, naphtyle, aralkyle en $C_7$-$C_{18}$ ou alkaryle en $C_7$-$C_{18}$ ou

$R^3$ signifie alkyle de 9 à 25 atomes de carbone, un groupe cycloalkyle de 5 à 12 atomes de carbone cycliques non substitué ou substitué par alkyle en $C_1$-$C_8$, un groupe (cycloalkyl en $C_5$-$C_{12}$)-(alkyle en $C_1$-$C_4$) non substitué ou substitué par alkyle en $C_1$-$C_8$, alcényle de 5 à 18 atomes de carbone, aralkyle en $C_7$-$C_{18}$ ou alkaryle en $C_7$-$C_{18}$ ou

$R^1$ et $R^3$ signifient alkyle de 1 à 25 atomes de carbone, un groupe cycloalkyle de 5 à 12 atomes de carbone cycliques non substitué ou substitué par alkyle en $C_1$-$C_8$, un groupe (cycloalkyl en $C_5$-$C_{12}$)-(alkyle en $C_1$-$C_4$) non substitué ou substitué par alkyle en $C_1$-$C_8$, alcényle de 2 à 18 atomes de carbone, phényle, naphtyle, aralkyle en $C_7$-$C_{18}$ ou alkaryle en $C_7$-$C_{18}$, qui sont à chaque fois substitués par un ou plusieurs groupes de la série halogène, cyano, nitro, -$OR^a$ ou -$COOR^b$, ou bien

$R^1$ et $R^3$ signifient alkyle de 2 à 25 atomes de carbone, un groupe cycloalkyle de 3 à 10 atomes de carbone cycliques non substitué ou substitué par alkyle en $C_1$-$C_8$, un groupe (cycloalkyl en $C_3$-$C_{12}$)-(alkyle en $C_1$-$C_4$) non substitué ou substitué par alkyle en $C_1$-$C_8$, alcényle de 3 à 18 atomes de carbone, aralkyle en $C_7$-$C_{18}$ ou alkaryle en $C_7$-$C_{18}$, qui sont interrompus à chaque fois par un ou plusieurs groupes de la série -O-, -S-, -NH-,

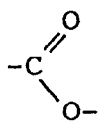 ou 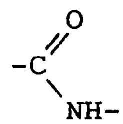 ,

les aralkyles en $C_7$-$C_{18}$ ou les alkaryles en $C_7$-$C_{18}$ n'étant interrompus que dans les restes alkyle, ou bien

$R^1$ et $R^3$ signifient alkyle de 2 à 25 atomes de carbone, un groupe cycloalkyle de 3 à 10 atomes de carbone cycliques non substitué ou substitué par alkyle en $C_1$-$C_8$, un groupe (cycloalkyl en $C_3$-$C_{12}$)-(alkyle en $C_1$-$C_4$) non substitué ou substitué par alkyle en $C_1$-$C_8$, alcényle de 3 à 18 atomes de carbone, aralkyle en $C_7$-$C_{18}$ ou alkaryle en $C_7$-$C_{18}$, qui sont à chaque fois substitués par un ou plusieurs groupes de la série halogène, cyano, nitro, -$OR^a$ ou -$COOR^b$ et sont interrompus à chaque fois par un ou plusieurs groupes de la série

-O-, -S-, -NH-,

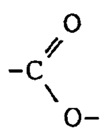 ou 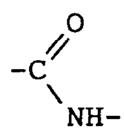 ,

les aralkyles en $C_7$-$C_{18}$ ou les alkaryles en $C_7$-$C_{18}$ n'étant interrompus que dans les restes alkyle,

où $R^a$ a la signification de $R^3$ à l'exception de -$OR^a$ et $R^b$ signifie alkyle de 1 à 18 atomes de carbone ou alkyle de 2 à 20 atomes de carbone interrompu par un ou plusieurs groupes de la série

-O-, -S-, -NH-,

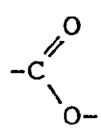 ou 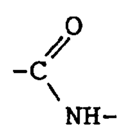 ,

ou un groupe cycloalkyle de 5 à 12 atomes de carbone cycliques non substitué ou substitué par alkyle en $C_1$-$C_8$, phényle, naphtyle, aralkyle en $C_7$-$C_{18}$ ou alkaryle en $C_7$-$C_{18}$, et

$R^6$ et $R^7$ signifient alkyle de 11 à 18 atomes de carbone, ou alkyle de 2 à 20 atomes de carbone qui est interrompu par un ou plusieurs groupes de la série -O-, -S-, -NH-,

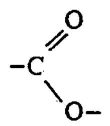 ou 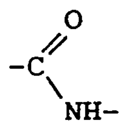 ,

ou un groupe cycloalkyle de 5 à 12 atomes de carbone cycliques non substitué ou substitué par alkyle en $C_1$-$C_8$, un groupe (cycloalkyl en $C_5$-$C_{12}$)-(alkyle en $C_1$-$C_4$) non substitué ou substitué par alkyle en $C_1$-$C_8$, ou bien un groupe (cycloalkyl en $C_3$-$C_{12}$)-(alkyle en $C_1$-$C_4$) non substitué ou substitué par alkyle en $C_1$-$C_8$, qui est interrompu par un ou plusieurs groupes de la série
-O-, -S-, -NH-,

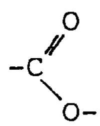 ou 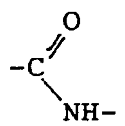 ,

ou alkaryle en $C_7$-$C_{18}$ ou aralkyle en $C_7$-$C_{18}$, ou bien,
quand x = 2,
R a la signification de $R^8$ et
$R^8$ signifie alkylène de 1 à 18 atomes de carbone, alkylène de 2 à 18 atomes de carbone qui est interrompu par au moins un groupe -O-, alkylidène de 2 à 20 atomes de carbone ou alkylidène de 3 à 20 atomes de carbone qui est interrompu par au moins un groupe -O-,
caractérisé en ce qu'on fait réagir un composé de formule

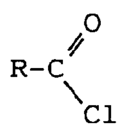

avec KSCN pour donner un composé de formule

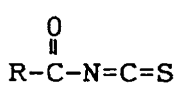

et on fait réagir le composé

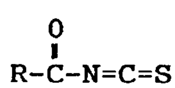

avec $HOR^6$ pour donner

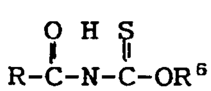

ou on fait réagir le composé

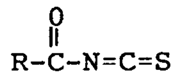

avec $HSR^7$ pour donner

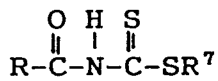

ou en ce qu'on fait réagir un composé de formule

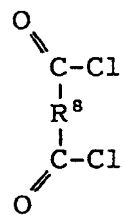

avec KSCN pour donner

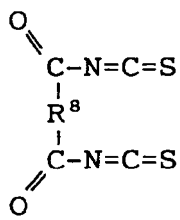

,

et on fait réagir ce composé avec 2 moles du composé $HR^2$ pour donner un composé de formule

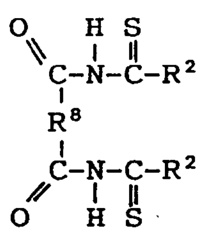

,

où R, $R^2$, $R^6$, $R^7$ et $R^8$ ont la signification indiquée ci-dessus.

**16.** Procédé de préparation de composés de formule IIa

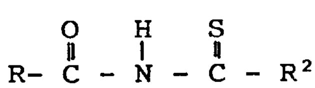 (IIa)

selon la revendication 15.

**17.** Procédé de préparation de composés de formule IIc

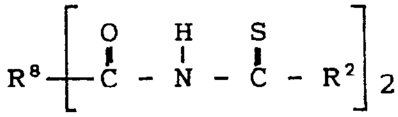

(IIc)

selon la revendication 15.

**18.** Procédé de préparation de composés de formule IIa dans laquelle R signifie $R_1$ ou -O-$R^3$ et $R^1$ est phényle ou alkyle de 1 à 18 atomes de carbone et $R^3$ est alkyle de 10 à 18 atomes de carbone ou phényle, et $R^2$ signifie -S$R^7$, selon la revendication 16.

**19.** Procédé de préparation de composés de formule IIa, dans laquelle $R^7$ signifie alkyle de 12 à 18 atomes de carbone, -$CH_2$-COO-(alkyle en $C_1$-$C_{18}$) ou phényl-(alkyle en $C_1$-$C_4$), selon la revendication 18.

**20.** Utilisation des composés de formule I selon la revendication 1 et de formule II selon la revendication 15 comme additifs d'extrême pression et anti-usure et comme antioxydants dans des lubrifiants et des fluides hydrauliques.